# EUROPEAN PATENT SPECIFICATION

(11) **EP 3 184 109 B1**
(45) Date of publication and mention of the grant of the patent: **18.11.2020**
(21) Application number: 17155337.3
(22) Date of filing: 29.12.2010
(51) Int. Cl.: A61K 31/455, A61K 45/06, A61K 35/17, C12N 5/0783, A61K 38/20

(54) **METHODS FOR ENHANCING NATURAL KILLER CELL PROLIFERATION AND ACTIVITY**
METHODEN ZUR VERSTÄRKUNG DES WACHSTUMS UND DER AKTIVIERUNG VON NK-ZELLEN
PROCÉDÉS D'AMPLIFICATION DE LA PROLIFÉRATION ET DE L'ACTIVITÉ DES CELLULES TUEUSES NATURELLES

(30) Priority: 29.12.2009 US 282196 P
(43) Date of publication of application: 28.06.2017
(62) Divisional of application: 10807391.7
(73) Proprietor: Gamida-Cell Ltd., 95484 Jerusalem (IL)
(72) Inventor: Peled, Tony, 9078719 Mevaseret Zion (IL); Frei, Gabi M., 9962145 Bet-Shemesh (IL)
(74) Representative: Gulde & Partner

(56) References cited:
- WO-A2-2006/050270
- WO-A2-2007/063545
- WO-A2-2008/056368
- US-A1- 2006 093 605
- OLSON JANELLE A ET AL: "Tissue-Specific Homing and Expansion of Donor NK Cells in Allogeneic Bone Marrow Transplantation", JOURNAL OF IMMUNOLOGY, vol. 183, no. 5, September 2009 (2009-09), pages 3219-3228, XP009147122, ISSN: 0022-1767
- VERNERIS MICHAEL R ET AL: "The phenotypic and functional characteristics of umbilical cord blood and peripheral blood natural killer cells", BRITISH JOURNAL OF HAEMATOLOGY, vol. 147, no. 2, October 2009 (2009-10), pages 185-191, XP009146910, ISSN: 0007-1048
- LI F ET AL: "Cell life versus cell longevity: The mysteries surrounding the NAD<+> precursor nicotinamide", CURRENT MEDICINAL CHEMISTRY, BENTHAM SCIENCE PUBLISHERS BV, BE, vol. 13, no. 8, 1 April 2006 (2006-04-01), pages 883-895, XP002539111, ISSN: 0929-8673, DOI: DOI:10.2174/092986706776361058
- ALICI E ET AL: "Autologous antitumor activity by NK cells expanded from myeloma patients using GMP-compliant components", BLOOD, AMERICAN SOCIETY OF HEMATOLOGY, US, vol. 111, no. 6, 15 March 2008 (2008-03-15), pages 3155-3162, XP003027014, ISSN: 0006-4971, DOI: DOI:10.1182/BLOOD-2007-09-110312
- PELED TONY ET AL: "Nicotinamide modulates ex-vivo expansion of cord blood derived CD34+cells cultured with cytokines and promotes their homing and engraftment in SCID mice", BLOOD, AMERICAN SOCIETY OF HEMATOLOGY, US, vol. 108, no. 11, Part 1, 1 November 2006 (2006-11-01), page 218A, XP009134130, ISSN: 0006-4971
- DE RIDDER MARK ET AL: "Hypoxic tumor cell radiosensitization: role of the iNOS/NO pathway", BULLETIN DU CANCER (MONTROUGE), vol. 95, no. 3, March 2008 (2008-03), pages 282-291, XP009146914, ISSN: 0007-4551

## Description

### FIELD AND BACKGROUND OF THE INVENTION

The present invention, in some embodiments thereof, relates to *ex-vivo* culture of natural killer (NK) cells and, more particularly, but not exclusively, to compositions and methods for enhancing propagation and/or functionality of NK cells by treating the cells with nicotinamide in combination with cytokines driving NK cell proliferation.

Natural killer (hereinafter also abbreviated as "NK") cells are lymphoid cells that participate in immune reactions. These cells have variety of functions, especially the killing of tumor cells, cells undergoing oncogenic transformation and other abnormal cells in a living body, and are important components of innate immunological surveillance mechanisms. Clinical experience with adoptive immunotherapy with NK cells has emphasized the need for better methods for effectively and efficiently expanding NK cell populations while maintaining, and even enhancing their functionality *in-vivo* (killing ability trafficking, localization, persistence and proliferation).

NK cells represent a distinct population of lymphocytes in terms of both phenotype and function. NK cells have a large granular lymphocyte morphology and express characteristic NK cell surface receptors, and lack both TCR rearrangement and T cell, B cell, monocyte and/or macrophage cell surface markers. The cells kill by releasing small cytoplasmic granules of proteins (perforin and granzyme) that cause the target cell to die by apoptosis. NK cells possess mechanisms distinguishing between potential "target" cells and healthy cells via a multitude of inhibitory and activating receptors that engage MHC class I molecules, MHC class I-like molecules, and molecules unrelated to MHC (Caliguiri Blood 2008 112:461-69). Inhibitory NK cell receptors include HLA-E (CD94/NKG2A); HLA-C (group 1 or 2), KIR2DL; KIR3DL (HLA-B Bw4) and HLA-A3 or A4 + peptide. Activating NK cell receptors include HLA-E (CD94/NKG2C); KIR2DS (HLA-C) and KIR3DS (HLA-Bw4). Other receptors include the NK cell receptor protein-1 (termed NK1.1 in mice) and the low affinity receptor for the Fc portion of IgG (FcγRIII; CD16). Specific NK cell activators, the UL binding proteins (ULBPs), and their potential therapeutic use are described in detail in US patent application US20090234699 to Cosman et al. "Activating" and "inhibitory" surface receptors control the NK cell's cytotoxic activity. Importantly for therapeutic considerations, NK cell inhibition is required to prevent destruction of normal host tissues by "activated" NK cells, but inhibitory signaling in NK cells appears to be stronger than the activating signals.

The intact bone marrow is necessary for NK cell generation. Human bone marrow-derived NK cells are large granular lymphocytes (LGL) of the CD2+CD16+CD56+ phenotype, lacking CD3 yet containing the T-cell receptor zeta-chain [zeta(ζ)-TCR]. NK cells can be found within a variety of lymphoid and nonlymphoid tissues, including blood, spleen, liver, lungs, intestines, and decidua. NK cells have been found in significant numbers in tumors, where they may exert antitumor activity.

NK cells exhibit spontaneous non-MHC-restricted cytotoxic activity against virally infected and tumor cells, and mediate resistance to viral infections and cancer development in vivo. Thus, NK cells represent major effector cells of innate immunity. In addition, NK cells possess a variety of other functions, including the ability to secrete cytokines and to regulate adaptive immune response and hemopoiesis. NK cells provide requisite interferon-gamma (IFN-gamma) during the early stages of infection in several experimental animal models.

Most cancers lack identifiable, tumor-specific antigens in the HLA context, and thus cannot succumb to antigen specific cytotoxic T lymphocytes. Since a wide range of cancer cells are sensitive to NK cytotoxicity, transplantation of natural killer (NK) cell can be employed against cancer cells in an allogeneic setting, without risk of graft-versus-host disease.

Recent studies have emphasized this potential of NK-cell therapy. In animal models of transplantation, donor NK cells lyse leukemic cells and host lympho-hematopoietic cells without affecting nonhematopoietic tissues. Because NK cells are inhibited by self-HLA molecules which bind to killer immunoglobulin-like receptors (KIR), these findings have led to the clinical practice of selecting hematopoietic stem cell transplant donors with an HLA and KIR type that favors NK-cell activation (HLA- and KIR mismatch) and thus could be expected to promote an antileukemic effect. However, selection of the "best" donor is limited to patients who have more than one potential donor and the capacity of NK cells to lyse lymphoid cells is generally low and difficult to predict. A survey of NK distribution and function in autoimmune conditions has indicated reduced numbers and functionality of the NK cell population in many autoimmune diseases (e.g., SLE, Sjogren's syndrome, sclerosis, psoriasis, RA, ulcerative colitis, etc). Thus, treatment with NK cells may actively suppress potentially pathogenic autoimmune T cells that can mediate the inflammatory responses following bone marrow transplant, regulating the activation of autoimmune memory T cells in an antigen non-specific fashion to maintain the clinical remission and prevent GVH effect.

For clinical use NK cells are usually collected from the patient or donor by leukapheresis. However, maximal NK-cell dose is limited and high NK-cell doses may only be obtained for patients with a low body weight, making children the best candidates for NK-cell therapy. Significantly, the total number and activity of NK cells may substantially decrease in viral infection and/or cancer, making immunotherapy based on the activation of endogenous NK cells ineffective. Further, refractory relapses are a major complication in cell transfusions, and many clinical protocols require repeated infusions of lymphocyte populations.

In this regard, Verneris et al. (Brit J Hematol 2009;147:185-91), reviewing the prospects for clinical use of cord blood NK cells, has recently indicated that fresh cord blood NK cell populations may require further manipulation in order to express their full functional (cytotoxic, motility) potential. Upon systemic treatment with various biologic response modifiers, particularly IL-2, the number of activated NK cells and their antiviral and antimetastatic activities have been found to increase dramatically in various tissues. Based on this evidence, therapeutic strategies involving activation and expansion of NK cells along with IL-2 (and IL-15) have been attempted, as well as coadministration of IL-2 to the transfusion recipient. However, to date the results have been disappointing, indicating only limited homing and transient engraftment of the infused NK cells. Further, IL-2 is toxic, and must be used with extreme caution in the clinical setting.

In an attempt to develop a clinical feasible protocol for enrichment and proliferation of NK cells *ex-vivo,* magnetic cell-selection technology, using paramagnetic CD56 microbeads and cell selection columns, has been used to isolate a CD56⁺ population containing both CD3⁻/56⁺ NK (60.6±10.8 %) and CD3⁺/56⁺ NK T cells (30.4±8.6 %) to initiate the proliferation studies. With the addition of recombinant human IL-2 or IL-2 plus recombinant human IL-15 substantial cell-expansion variability was observed, depending on the donor, and even when the same donor was tested on different occasions. The cytotoxicity of selected and propagated CD56⁺ cells at a low E:T ratio was significantly higher than the starting population, but was comparable to non-separated PBMC cultured for 2 weeks under the same conditions. In fresh, unselected PBMC cultures, IL-15 (in combination with IL-2) induced higher killing at the 1:1 E:T ratio than IL-2 alone. Notably, since CD3+ cells were not depleted prior to culture, the proliferation of CD3⁺CD56⁺ NKT cells was 2-3 times that of CD3⁻CD56⁺ NK cells. Only moderate proliferation of CD56⁺/CD3⁻ cells occurred, with the majority of the resultant cells being CD56⁺/CD3⁺ NKT cells.

In a different approach, human CD3-CD56+ NK cells are cultured from BM-derived CD34+ hematopoietic progenitor cells (HPCs) cultured in the presence of various cytokines produced by bone marrow stromal cells and/or immune cells (such as c-*kit* ligand, IL-2, and IL-15). The addition of the stem cell factor to these cultures has no effect on the differentiation of the CD3-CD56+ cytotoxic effector cells, but greatly enhances their proliferation in culture. The majority of these cells lack CD2 and CD16, but do express zeta-TCR. Similar to NK cells found in peripheral blood, bone marrow derived CD2-CD16-CD56+ NK cells grown in the presence of IL-15 were found to be potent producers of IFN-gamma in response to monocyte-derived cytokines. IL-15 can induce CD34+ HPCs to differentiate into CD3-CD56+ NK cells, and KL can amplify their numbers. However, yields of NK cells are limited by the low numbers of potential NK progenitors among the CD34+ cell population.

Other methods for the propagation of NK cells have been described. Frias et al. (Exp Hematol 2008; 36: 61-68) grew NK progenitors (CD7⁺CD34⁻Lin⁻CD56⁻) selected from cord blood on stromal cell layers with a serum-free medium, inducing NK differentiation with SCF, IL-7, IL-15, FL and IL-2, producing increased numbers of cytotoxic cultured NK cells. Harada et al. (Exp Hematol. 2004;32:614-21) grew NK cells on cells from a Wilm's tumor cells line. Waldmann et al. (US20070160578) describes enhanced proliferation of NK and CD8-T cells from whole blood, bone marrow or spleen cells in culture using complexes of IL-15/R-ligand activator, in order to reduce undesirable cytokine production. Campana et al. (US20090011498) describes *ex-vivo* culture and activation of NK cells, for transplantation, in the presence of leukemia cells expressing IL-15 and 4-1BB, and having weak or absent MHC-I or II expression. Childs et al. (US20090104170) describes *ex-vivo* proliferation, and activation of NK cells by co-culture with irradiated EBV-transformed lymphoblastoid cells, in the presence of IL-2. Using another approach, Tsai (US20070048290) produced continuous NK cell lines from hematopoietic stem cells by *ex-vivo* culture of immortalized NK-progenitors with irradiated 3T3-derived OP-9S cells, for research and potential therapeutic applications.

However, established methods for NK cell culture also support T cell proliferation and even after T cells are depleted, residual T cells typically increase in number after stimulation, precluding clinical use of the expanded cell populations due to potential graft versus host disease. This further necessitates another round of T cell depletion before infusion, making the preparatory procedure time consuming, expensive and invariably causing substantial NK cell loss.

To reduce T cell contamination following expansion, NK expansion protocols are using purified CD56+CD3- cells as the initial population to be seeded in expansion cultures. To obtain a highly purified fraction of CD56+CD3- cells, a two step purification procedure is needed: positive selection of CD56 cells followed by depletion of CD3+ cells or first the depletion of the CD3 cells followed by positive selection of CD56 cells. However, this procedure is expensive and involves a substantial cell lost during the two cycle of purification. Even in cultures initiated with purified CD56+CD3- cells there are still expanded NK products contaminated with T cells.

Protocols using cytokines only for the expansion of NK cells indicate a rather modest effect and the requirement for additional stimuli in addition to cytokines in order to obtain substantial expansion (Korean J Lab Med 2009;29:89-96, Koehl U et al. Klin Pädiatr 2005; 217: 345-350). Irradiated feeder cells (e.g., peripheral blood mononuclear cells, Epstein-Barr virus-transformed lymphoblastoid lines (ABV-LCL), K562 myeloid leukemia cell line, genetically modified to express a membrane-bound form of interleukin-15 and the ligand for the co-stimulatory molecule 4-1BB) and others are commonly used for the expansion of NK cells as additional stimuli. While most NK expansion protocols use purified CD56+CD3- cells as the initial population, some protocols use mononuclear cells as the initial seeding population in combination with irradiated stroma or anti-CD3 antibody (Blood, 15 March 2008, Vol. 111, No. 6, pp. 3155-3162). Following expansion these cultures are heavily contaminated with CD3+ and CD3+CD56+ cells and therefore CD56+CD3- cells need to be purified before infusion.

Miller at al (Blood, 1992 80: 2221-2229) obtained a 30-fold expansion of NK cells at 18 days culture using a fraction enriched for NK progenitors and monocytes comprising CD56+CD3- cells in combination with purified CD14+ cells or MNC depleted of CD5 and CD8 by panning on antibody-coated plastic flasks. Ve'ronique Decot et al. (Experimental Hematology 2010;38:351-362) reported about 20 fold expansion of NK cells on irradiated T and B cells by depleting mononuclear cells of T and B, and found that the contaminating population after depletion was mainly monocytes. However, in this culture model, feeder cells and cytokines were necessary to obtain NK cell amplification because no expansion was observed in the presence of cytokines alone or feeder cells alone. Therefore, in contrast to Miller, even thought monocytes were enriched in the seeding population, no expansion of NK cells was observed in the absence of irradiated T and B stroma cells (Decot et al., Exper Hematology 2010;38:351-362).

Yet further, while *ex-vivo* cultured NK cells often demonstrate considerable activity (e.g., cytotoxicity) against unrelated target cells, activity against more clinically relevant tumor and cancer cells, both *in-vitro* and *in-vivo* has often been disappointing, and methods for enhancing activation have been proposed. Zitvogel et al. (US Patent No. 6,849,452) teaches *ex-vivo* or *in-vivo* activation of NK cells by contacting with triggered dendritic cells. Others have suggested enhancing activation by culturing NK cells with cells lacking MHC-I molecules and genetically modified to express IL-15 (Campana et al., US Patent Application No. 2009011498) or pre-treatment of NK cell recipients with proteasome inhibitors (Berg et al. Cytotherapy 2009; 11:341-55). However, none of the protocols have yielded significantly expanded NK cell populations capable of survival and expansion in appropriate host target organs following transplantation (homeostatic proliferation) and immunotherapy with ex vivo proliferated NK cells is still limited by the inability to obtain sufficient numbers of highly purified, functionally competent NK cells suitable for use in clinical protocols (see Bachanova et al., Canc Immunol. Immunother. 2010; 59:739-44; Guven, Karolinska Institute, 2005; Schuster et al., E.J. Immunology 2009;34:2981-90; Bernardini et al. Blood 2008; 111:3626-34). Thus there is a need for simplified, cost-effective methods to preferentially propagate NK *ex-vivo,* as isolated NK cells, or from a mixed population of mononuclear cells either depleted or not from CD3+ cells.

It was shown that nicotinamide can modulate cell fate and function (see, for example WO 2003/062369, WO 2005/007799, WO 2005/007073, WO 2006/030442, WO 2007/063545 and WO 2008/056368).

### SUMMARY OF THE INVENTION

In view of the growing need for greater numbers of therapeutically competent NK cells for clinical applications such as cell therapy for leukemia and other cancers, there is a need for improved, simplified and cost-effective methods for enhanced *ex-vivo* proliferation and activation of natural killer cells suitable for use in the clinical setting.

Thus, expansion of NK cells in *ex vivo* cultures, and enhancing their functionality following infusion is critical to their clinical applicability in adoptive immunotherapy.

The present invention provides for subject matter as defined in the appended claims.

According to an aspect of some embodiments of the present invention there is provided a method of *ex-vivo* culturing natural killer (NK) cells as described in the claims, the method comprising culturing a population of cells comprising NK cells with at least one growth factor and an effective concentration, effective exposure time and effective duration of exposure of nicotinamide, wherein culturing the NK cells with the at least one growth factor and the effective concentration, effective exposure time and effective duration of the nicotinamide results in at least one of the following:
(a) elevated expression of CD62L as compared to NK cells cultured under otherwise identical culturing conditions with less than 0.1 mM of the nicotinamide; and
(b) increased cytotoxic activity as compared to NK cells cultured under otherwise identical culturing conditions with less than 0.1 mM of the nicotinamide.

Further, there is disclosed a population of NK cells cultured according to the method of the present invention.

Accordingly, there is disclosed a population of NK cells characterized by at least one of the following:
(a) elevated expression of CD62L as compared to a population of NK cells cultured under otherwise identical culturing conditions with less than 0.1 mM of the nicotinamide
(b) elevated migration response as compared to a population of NK cells cultured under otherwise identical culturing conditions with less than 0.1 mM of the nicotinamide;
(c) elevated homing and in-vivo retention as compared to a population of NK cells cultured under otherwise identical culturing conditions with less than 0.1 mM of the nicotinamide;
(d) greater proliferation as compared to a population of NK cells cultured under otherwise identical culturing conditions with less than 0.1 mM of the nicotinamide;
(e) increased cytotoxic activity as compared to a population of NK cells cultured under otherwise identical culturing conditions with less than 0.1 mM of the nicotinamide;
(f) a reduced ratio of CD3+ to CD56+/CD3- cells as compared to a population of NK cells cultured under otherwise identical culturing conditions with less than 0.1 mM of the nicotinamide.

There is further disclosed a population of NK cells characterized by enhanced homing, engraftment and retention when transplanted, wherein infusion of at least 15X10⁶ of the NK cell population into an irradiated SCID mouse host, results in at least 25 % donor-derived NK cells in a host lymphatic tissue, as detected by immunodetection and flow cytometry, at 4 days post infusion.

According to some embodiments disclosed herein the population of NK cells is further characterized by expression of CD62L in at least 30 % of said cell population at the time of infusion, as detected by immunodetection and flow cytometry. According to yet other embodiments disclosed herein, the population of NK cells is further characterized by a ratio of CD3+ to CD56+/CD3- cells equal to or less than 1:100 at the time of infusion.

According to still another aspect of some embodiments disclosed herein there is provided a method of inhibiting tumor growth in a subject in need thereof, comprising administering a therapeutically effective amount of the population of NK cells of the invention to the subject.

According to still another aspect of some embodiments disclosed herein there is provided a method of treating or preventing a viral infection in a subject in need thereof, comprising administering a therapeutically effective amount of the *ex-vivo* cultured population of NK cells of the invention to the subject.

According to another aspect of some embodiments disclosed herein there is provided a method of treating or preventing graft versus host disease in a subject in need thereof, comprising administering a therapeutically effective amount of the *ex-vivo* cultured population of NK cells of thee invention to the subject.

According to another aspect of some embodiments disclosed herein there is provided a method of treating or preventing an autoimmune disease or condition in a subject in need thereof, comprising administering a therapeutically effective amount of the *ex-vivo* cultured population of NK cells of the invention to the subject.

According to another aspect of some embodiments disclosed herein there is provided a method of treating or preventing a leukemic disease or condition in a subject in need thereof, comprising administering a therapeutically effective amount of the *ex-vivo* cultured population of NK cells of the invention to the subject.

According to some embodiments disclosed herein, the population of NK cells is autologous or allogeneic to the subject.

According to some embodiments disclosed herein the administering is by a single infusion or repeated infusions of the NK cell population.

According to some embodiments disclosed herein the subject is being treated with at one growth factor concomitantly with the administering of the NK cell population.

According to some embodiments of the present invention the at least one growth factor is IL-15.

According to another aspect of some embodiments of the present invention there is provided a method of transducing *ex-vivo* cultured NK cells with an exogene as defined in the claims, the method comprising:
(a) *ex-vivo* culturing a population of NK cells according to the method the invention; and
(b) transducing the cultured population of NK cells with the exogene.

According to some embodiments disclosed herein the at least one growth factor is IL-2, the exposure time is from seeding of the population of cells comprising NK cells, the exposure duration is from about 2 to about 3 weeks and the concentration of the nicotinamide is 5 mM.

According to some embodiments disclosed herein the effective concentration of the nicotinamide is about 0.5 mM to about 50 mM, or about 1.0 mM to about 25 mM, or about 2.5 mM to about 10 mM, or about 2.5 mM, about 5.0 mM or about 7.5 mM.

According to some embodiments disclosed herein the exposure time is from seeding to about 5 weeks after culturing, or from about 1 hour after seeding to about 3 weeks after culturing, or from about 24 hours after seeding to about 3 weeks after culturing, or from about 2 days after seeding to about 2 weeks after culturing, or from seeding of the population of cells comprising said NK cells in said culture.

According to some embodiments disclosed herein the exposure duration is from about 2 hours to about 5 weeks, or from about 30 hours to about 4 weeks, or from about 2 days to about 3 weeks, or about 1 week, about 2 weeks, about 3 weeks, about one day, about two days, about three days, about five days, about 10 days, about 12 days, about 15 days, about 17 days or about 20 days.

According to some embodiments of the present invention the population of cells comprising said NK cells is obtained from a source selected from the group consisting of cord blood, bone marrow and peripheral blood.

According to some embodiments of the present invention the population of cells comprising the NK cells is a heterogenous cell population which comprises an NK cell fraction and a CD3+ cell fraction.

According to some embodiments of the present invention the CD3+ cell fraction is greater than said NK cell fraction.

According to some embodiments of the present invention the NK cell fraction is greater than said CD3+ cell fraction.

According to some embodiments of the present invention the population of cells comprising the NK cells is a mononuclear or total nuclear cell population depleted of CD3+ cells.

According to some embodiments of the present invention the population of cells comprising the NK cells is a mononuclear or total nuclear cell population depleted of CD3+ and CD19+ cells.

According to some embodiments of the present invention the population of cells comprising the NK cells is an unselected NK cell population.

According to some embodiments of the present invention the NK cells comprise CD56+CD3- cells.

According to some embodiments of the present invention the NK cells comprise CD56+CD16+CD3- cells.

According to some embodiments of the present invention, culturing the population of cells comprising the NK cells is effected without a feeder layer or feeder cells.

According to some embodiments of the present invention the at least one growth factor is IL-15.

According to some embodiments of the present invention the expression of CD62L is determined by a method selected from the group consisting of flow cytometry, immunodetection, quantitative cDNA amplification and hybridization.

According to some embodiments of the present invention the expression of CD62L is determined by fluorescent activated cell sorting (FACS).

According to some embodiments of the present invention the expression of CD62L is determined is using fluorescent anti-human CD62L monoclonal antibodies.

According to some embodiments of the present invention the elevated migration response is determined by a transmigration or gap closure assay.

According to some embodiments of the present invention the elevated migration response is determined by a transmigration assay.

According to some embodiments of the present invention the transmigration is assayed in response to stimulation with SDF1.

According to some embodiments of the present invention the elevated homing and *in-vivo* retention is determined by FACS, expressed as percent engrafted NK cells in target organs following infusion.

According to some embodiments of the present invention the target organ is selected from the group consisting of spleen, bone marrow and lymph nodes.

According to some embodiments of the present invention the homing and engraftment is determined about 1 day to about 2 weeks following infusion of NK cells.

According to some embodiments of the present invention the proliferation rate is determined by clonogenic assays, mechanical assays, metabolic assays, and direct proliferation assays.

According to some embodiments of the present invention the proliferation rate is determined by FACS analysis of percentage CD56+CD3- cells and expressed as fold increase over time.

According to some embodiments of the present invention the cytotoxic activity is assayed using a cell killing assay.

According to some embodiments of the present invention the target cells of the cell killing assay are a cancer cell line, primary cancer cells, solid tumor cells, leukemic cells or virally infected cells.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

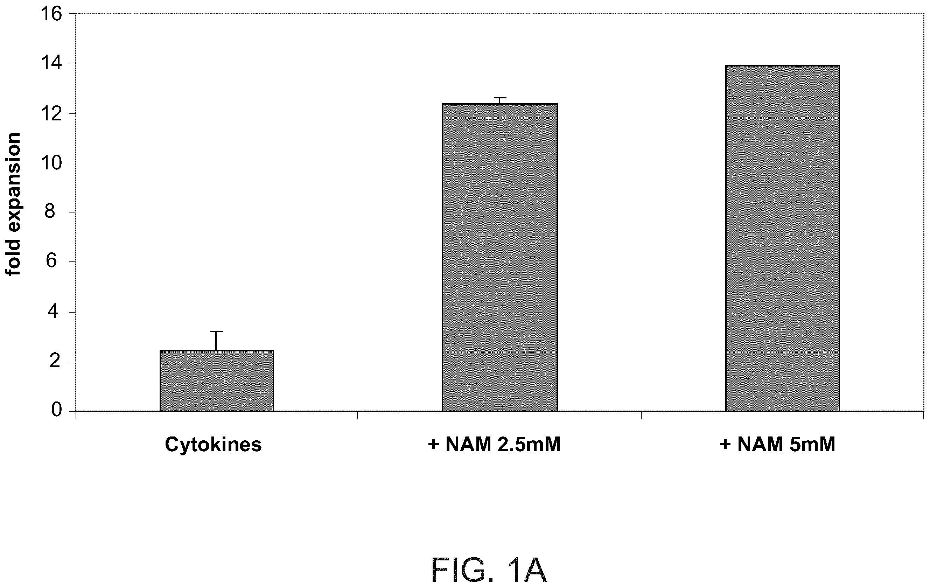

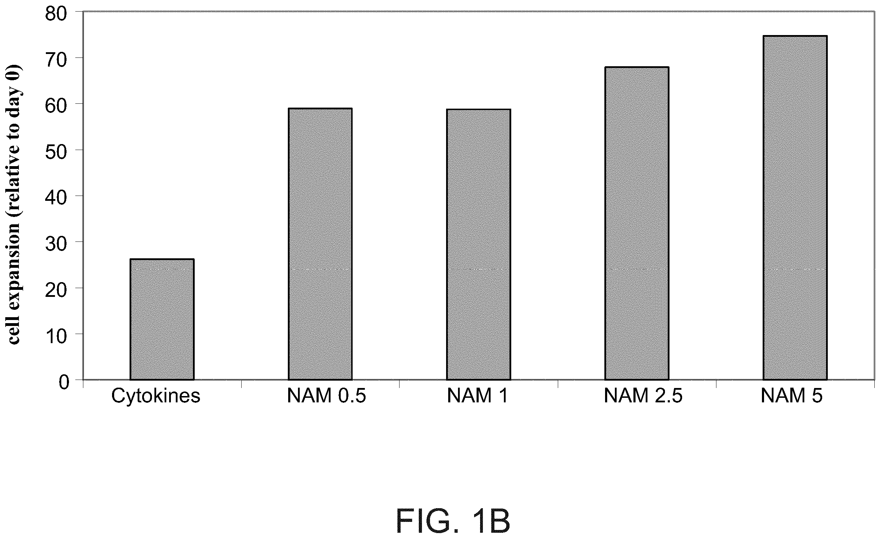

FIGs. 1A and 1B are histograms showing the dose-dependent proliferation of cord-blood derived, purified NK cell fraction after culturing in the absence (cytokines) or presence of increasing concentrations (0.5 to 5 mM "NAM", as indicated) of nicotinamide. Cultures were initiated with cord blood NK cells purified on immunomagnetic beads for CD56+ phenotype, and maintained with cytokines (including Flt-3, IL-2 and IL-15) for up to 3 weeks. FIG. 1A shows the proliferation (fold increase or "cell expansion", relative to day 0, initiation of the cultures) of the CD56+ NK cells at 14 days culture. FIG. 1B shows the fold increase of the CD56+ NK cells after 3 weeks culture. Note the dramatic dose-dependent increase in the CD56+ cell component of the nicotinamide treated cultures, continuing throughout the entire culture period, compared to controls (cytokines).

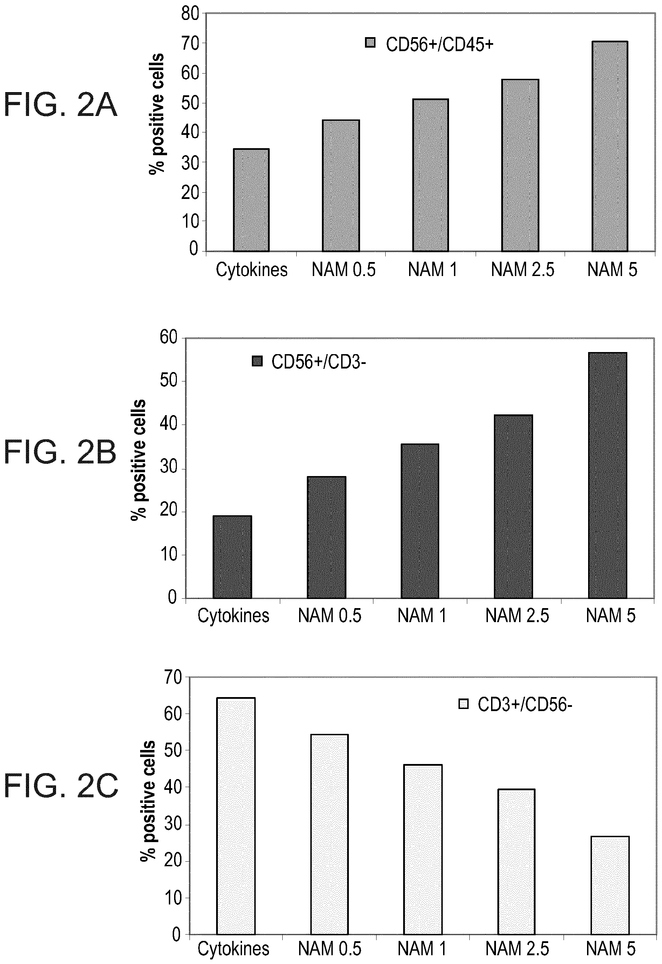

FIGs. 2A-2C are histograms showing the proportion of different lymphocyte subsets, grouped according to cell surface markers (CD56, CD45, CD3) in cultures initiated with the entire cord-blood-derived mononuclear cell fraction maintained in the absence (cytokines) or presence of nicotinamide. Cultures were maintained with cytokines (including Flt-3, IL-15 and IL-2), with or without increasing concentrations (0.5 to 5 mM) of nicotinamide ("NAM") for 3 weeks, reacted with specific antibodies for surface markers, and then analyzed by FACS for specific phenotypes. FIG. 2A = CD56+/CD45+ cells;(NK and NK-T cells) FIG. 2B = CD56+/CD3- (NK)cells; FIG. 2C = CD3+/CD56- (T) cells. Note the dose dependent increase in the NK cell population (CD56+/CD45+ and CD56+/CD3- phenotype), and concomitant decrease in the T lymphocyte population (CD3+/CD56- phenotype) in the nicotinamide treated cultures;

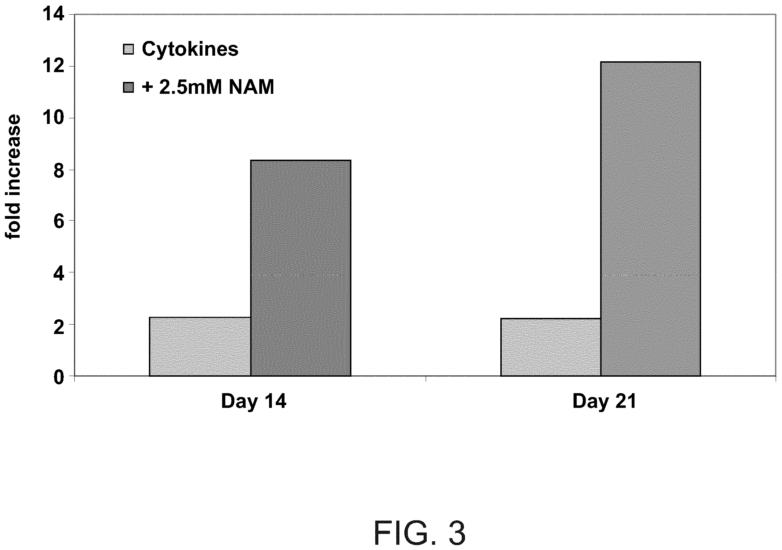

FIG. 3 is a histogram showing the proliferation of purified bone marrow derived, purified CD56+ cell fraction after culturing in the absence (cytokines) or presence of 2.5 mM nicotinamide. Cultures were initiated with CD56+ cells (NK and NK-T) purified from bone marrow on immunomagnetic beads, and maintained with cytokines (including Flt-3, IL-2 and IL-15) for up to 3 weeks with (dark shading) or without (cytokines, light shading) 2.5 mM nicotinamide. Note the dramatic increase in the CD56+CD3- NK cell component of the nicotinamide treated cultures, continuing throughout the entire culture period, compared to controls (cytokines, light shading);

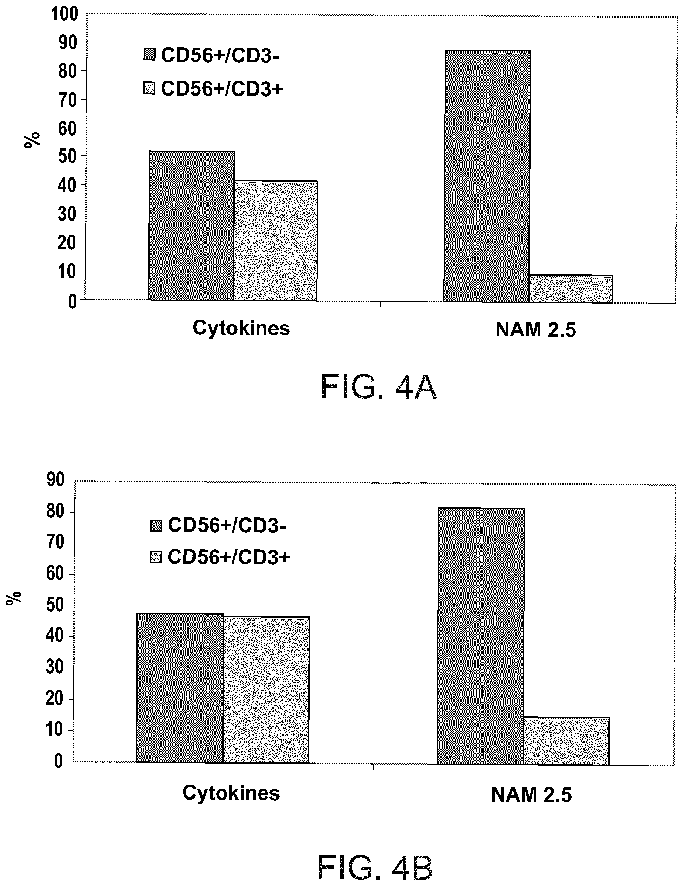

FIGs. 4A and 4B are histograms showing the proliferation of bone marrow NK vs NKT cells from purified bone marrow CD56+ cells after culturing in the absence (cytokines) or presence of nicotinamide. Cultures were initiated with bone marrow derived CD56+ cells purified on immunomagnetic beads, and maintained with cytokines (including Flt-3, IL-2 and IL-15) for 3 weeks, with or without 2.5 mM nicotinamide. FIGs. 4A and 4B represent the results of two independent exemplary experiments. Note the dramatic increase in the proportion of CD56+CD3- NK cells (dark shading), and reduction of CD56+CD3+ NKT cell (light shading) component of the nicotinamide treated cultures compared to controls (cytokines), independent of the initial proportion of NK vs NKT cells in the seeded cells;

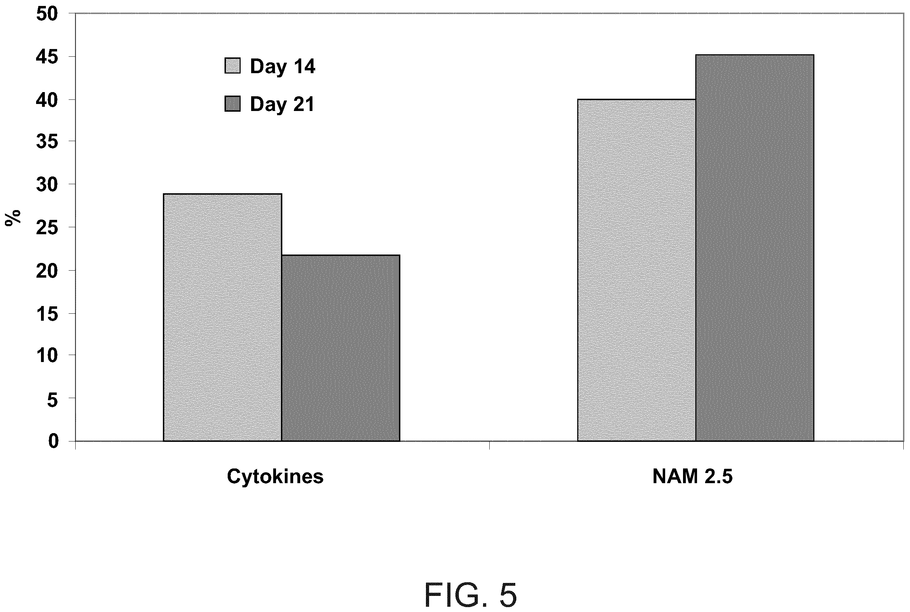

FIG. 5 is a histogram showing increased percentage of cells displaying the CD56+CD16+ NK cell phenotype from purified bone marrow CD56+ cells after culturing in the absence (cytokines) or presence nicotinamide. Cultures were initiated with bone marrow derived immunomagnetic purified CD56+ cells as described in FIGs. 4A and 4B and maintained with cytokines (including Flt-3, IL-2 and IL-15) with or without of 2.5 mM nicotinamide for up to 3 weeks. Note the increase in the proportion of CD56+CD16+ NK at 14 days (light shading), continuing still at 21 days (dark shading) in the nicotinamide treated cultures compared to the reduction of CD56+CD16+ cell fraction in the controls (cytokines);

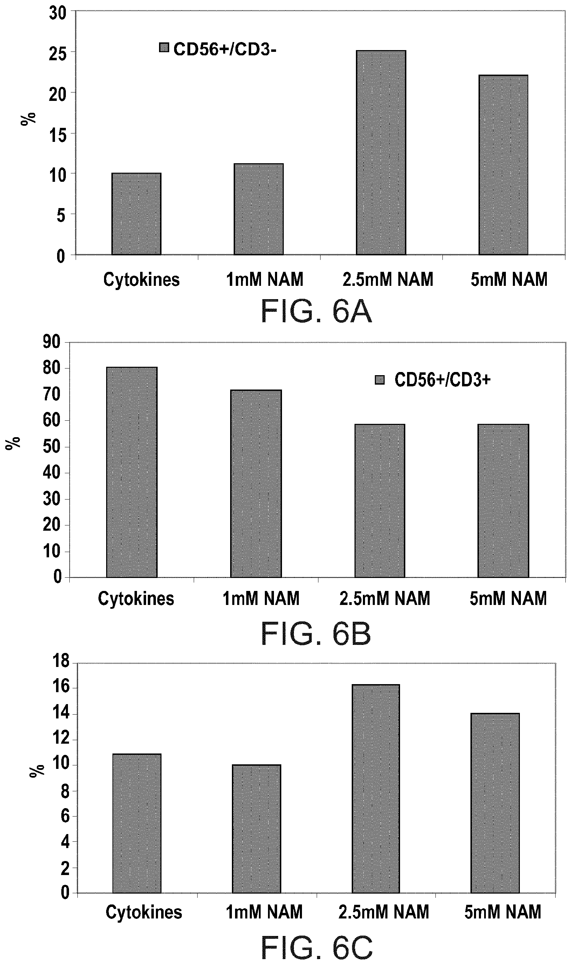

FIGs. 6A-6C are histograms showing the effect of short term culture with nicotinamide on proliferation of bone marrow NK and NKT cell subsets. Cultures were initiated with bone marrow-derived immunomagnetic purified CD56+ cells and maintained with cytokines (including Flt-3, IL-2 and IL-15) for 7 days in the absence (cytokines) or presence of increasing concentrations of nicotinamide ("NAM", 1 mM, 2.5 mM and 5 mM), and reacted with specific antibodies for surface markers, and analyzed by FACS for specific phenotypes. FIG. 6A represents percent of CD56+CD3-NK cell population as a function of nicotinamide; FIG. 6B depicts the reduction in the NKT cell CD56+CD3+ population as a function of nicotinamide in culture, and FIG. 6C represents the proliferation of the CD56+CD16+ NK cell subset as a function of nicotinamide, compared to controls (cytokines);

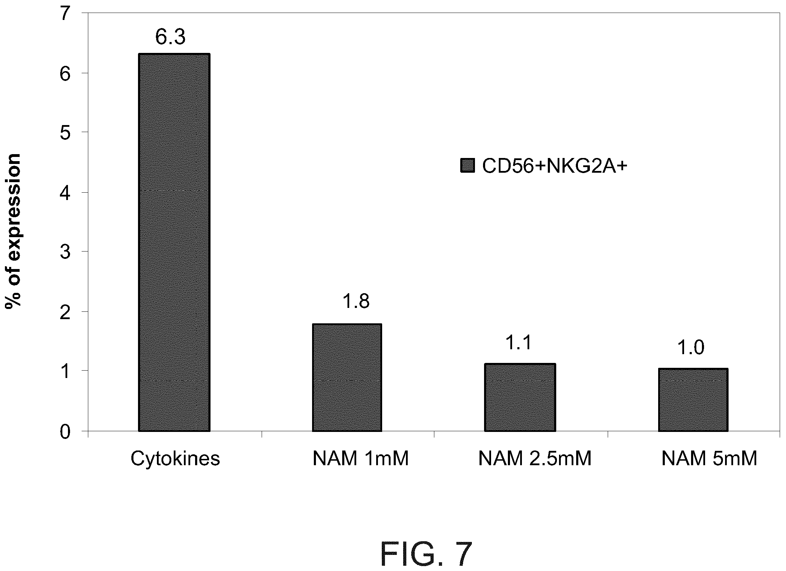

FIG. 7 is a histogram showing the reduction in the inhibitory NK cell CD56+/NKG2A+ subset of purified cord-blood derived CD56+ cells cultured for three weeks in the presence of increasing concentrations (1.0 to 5 mM) of nicotinamide. Immunomagnetic purified cord-blood-derived CD56+ cells were cultured with cytokines (including Flt-3, IL-2 and IL-15) in the presence or absence (cytokines) of increasing concentrations (1.0 to 5 mM) of nicotinamide ("NAM"). After three weeks the cells were reacted with specific antibodies for surface markers, and analyzed by FACS for the CD56+/NKG2A+ phenotypes. Dramatic reduction of CD56+NKG2A+ cells in the nicotinamide treated cultures, compared to controls (cytokines), suggests enhanced activation of NK cells with nicotinamide exposure;

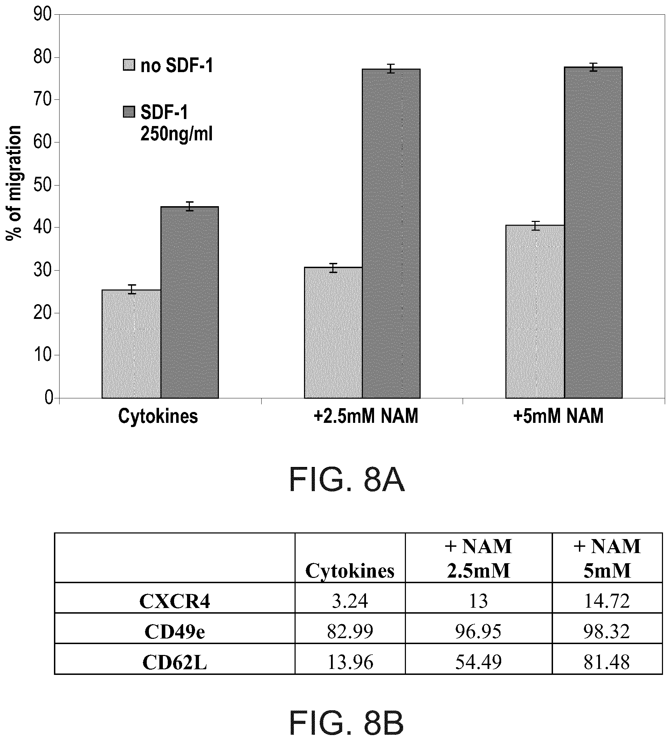

FIG. 8A is a histogram showing the enhanced migration potential of purified cord-blood derived NK cells cultured with increasing concentrations of nicotinamide. Immunomagnetic purified cord-blood-derived CD56+ cells were cultured with cytokines (including Flt-3, IL-2 and IL-15) in the absence (cytokines) or presence of 2.5mM or 5 mM nicotinamide. After two weeks cells were assayed for *ex-vivo* migration in response to 250 ng/ml SDF in a Transwell assay. Cells in the bottom chamber were counted by FACS. Enhanced migration, in the presence (dark shading) and absence (light shading) of SDF (250 ng/ml), compared to controls (cytokines), suggests enhanced motility and directed migration of NK cells by nicotinamide;
FIG. 8B is a table showing the increase in the expression of migratory (CXCR4), adhesion (CD49e) and trafficking (CD62L) receptors on cord-blood derived CD56+ cells cultured for three weeks in the presence of 2.5 or 5 mM of nicotinamide. Cultures were initiated with immunomagnetic beads purified cord-blood-derived CD56+ cells and maintained with cytokines (including Flt-3, IL-2 and IL-15) or cytokines plus nicotinamide (2.5 and 5 mM). After 3 weeks, cultured cells were reacted with specific antibodies for the specified surface markers, and then monitored by FACS. Note the dramatically enhanced expression of CXCR4 and CD62L, and elevated expression of CD49e in cells cultured in the presence of nicotinamide compared to controls (cytokines only);

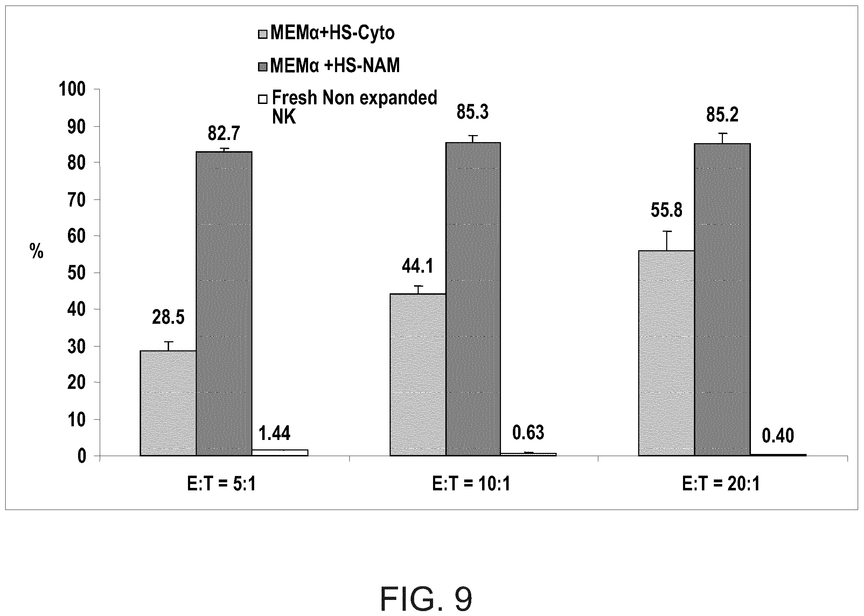

FIG. 9 is a histogram showing the enhanced killing potential of cord-blood derived CD56+ cells cultured with increasing concentrations of nicotinamide. Immunomagnetic beads purified cord-blood-derived CD56+ cells were cultured with cytokines (including Flt-3, IL-2 and IL-15), with (dark shading) or without (light shading) 2.5 mM nicotinamide. After 2 weeks FACScalibur analysis indicated that all cells had a CD56+/CD3- phenotype. The cord blood NK cells were assayed for *ex-vivo* killing potential with 5X10³ K562 target cells per assay, at 5:1, 10:1 and 20:1 NK cells per target cell (E:T). K562 cell death was monitored by FACS as a percentage of PI-positive CFSE-labeled cells. Enhanced target cell killing, compared to controls (cytokines) and fresh, non-cultured cord blood derived CD56+ cells (no shading) strongly suggests enhanced activation of NK cells killing potential by nicotinamide;

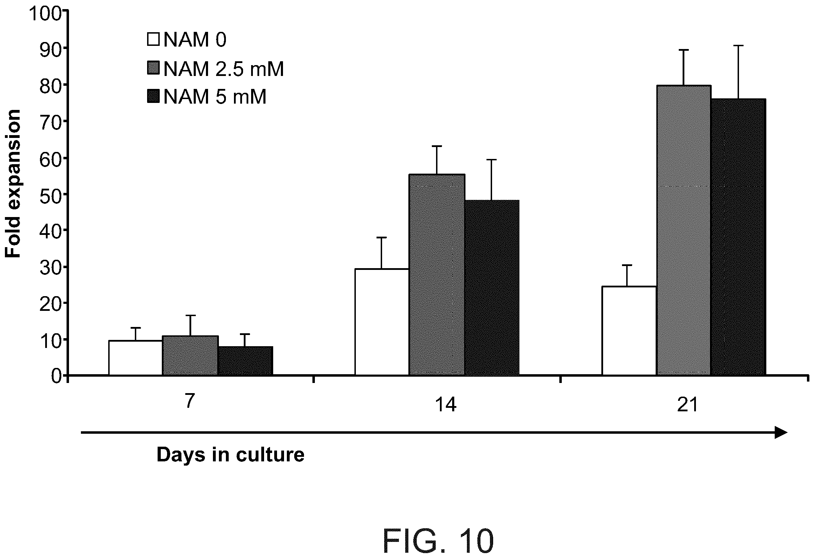

FIG. 10 is a histogram illustrating the expansion of human peripheral blood NK cells over three weeks culture with nicotinamide. Peripheral blood NK cells prepared by T-cell (CD3+ or CD3+CD19+) depletion of the mononuclear cells fraction of fresh units [MidiMACS isolation (MACS separation column, Cat. No.130-042-901) or RosetteSep Human CD3+ Cell Depletion Cocktail (Stem Cell Technologies, RosestteSep, Cat. No.15661)] were characterized by FACS analysis, and cultured in VueLife Bags in the presence of the indicated concentrations of nicotinamide (NAM 2.5 light shading and NAM 5mM dark shading). Control= cytokines only (NAM 0, no shading). Culture medium contained MEMα, Human Serum (10 %v/v) and cytokines (20ng/ml IL-15 and 50ng/ml IL-2 or only 50ng/ml IL-2). Culture volume was doubled after 1 and 2 weeks and the cells were counted and stained for FACS analysis after 7, 14 and 21 days. Note the greatly increased expansion (fold increase relative to day 0) in the presence of nicotinamide, while cytokines-only (NAM 0) controls show tendency to lose self-renewal capacity over time;

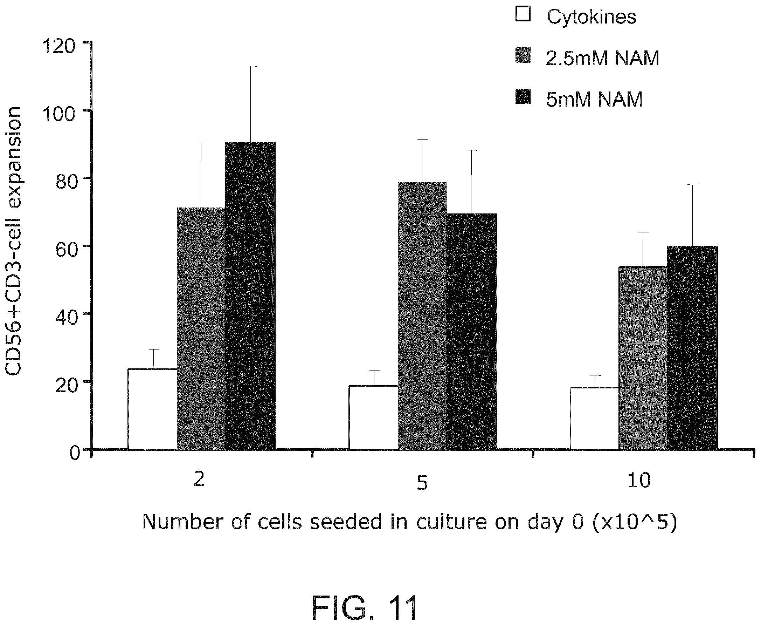

FIG. 11 is a histogram showing the effect of nicotinamide and seeding density on human peripheral blood NK cells over three weeks culture. Peripheral blood NK cells were prepared by T-cell (CD3+ or CD3+CD19+) depletion of the mononuclear cells as detailed in FIG. 10, and seeded at 2, 5 or 10X10⁵ cells/ml with 10 ml in each culture bag. Cells were then expanded in the presence of indicated concentrations (NAM 2.5, light shading and NAM 5mM, dark shading) of nicotinamide, or cytokines only (cytokines, no shading). Enhancement of NK cell proliferation by nicotinamide is evident at all seeding densities;

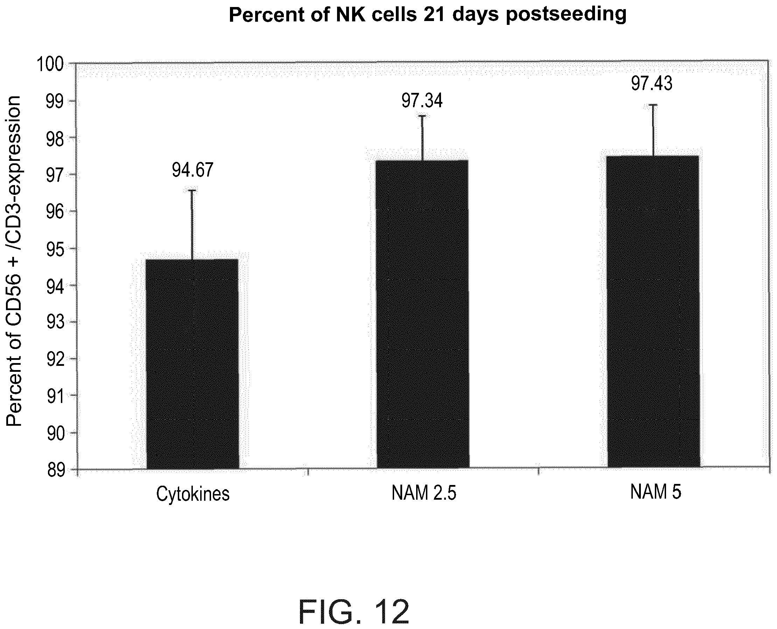

FIG. 12 is a histogram showing the percentages of CD56+CD3- NK cells in 21 day cultures of human peripheral blood NK cells. Peripheral blood NK cells were prepared by T-cell (CD3+ or CD3+CD19+) depletion of the mononuclear cells as detailed in FIG. 10, and seeded at 5 X10⁵ cells/ml with 10 ml in each culture bag. Cells were then cultured in the presence of indicated concentrations (NAM 2.5 and NAM 5mM) of nicotinamide, or cytokines only (cytokines). Note that even though percentages of NK cells were higher in all groups after 21 days in culture, in cultures exposed to nicotinamide the NK percentages are even higher than in control cultures;

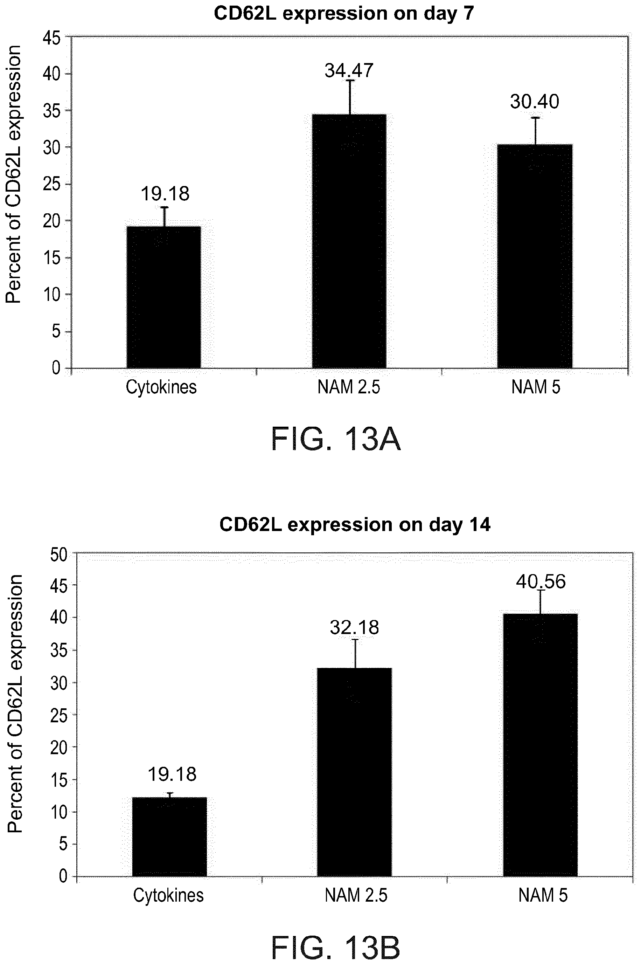

FIGs. 13A-13C are histograms showing the increased expression of the migratory receptor CD62L in cultures of human peripheral blood NK cells exposed to nicotinamide. Peripheral blood NK cells were prepared by T-cell (CD3+ or CD3+CD19+) depletion of the mononuclear cells as detailed in FIG. 10, and expanded in the presence of indicated concentrations (NAM 2.5 and NAM 5mM) of nicotinamide, or cytokines only (cytokines). After 7 (13A), 14 (13B) and 21 (13C) days, cultured cells were reacted with specific antibodies for the specified surface markers, and then monitored by FACS. Note the dramatically enhanced expression of CD62L, increasing with duration of exposure, in cells cultured in the presence of nicotinamide compared to controls (cytokines only);

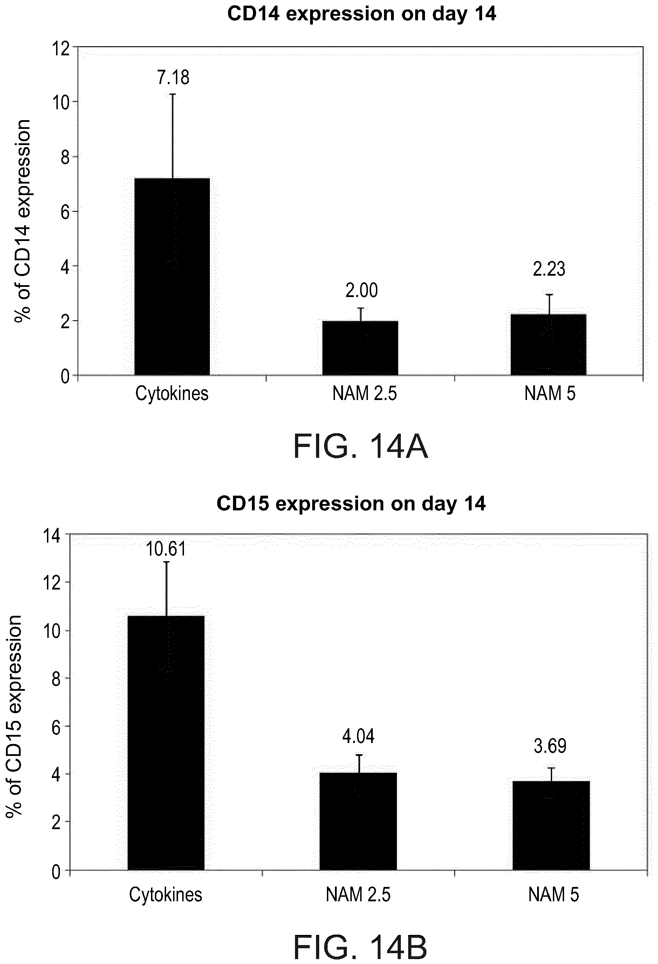

FIGs. 14A-14B are histograms showing inhibition of monocyte and granulocyte proliferation in cultures of human peripheral blood NK cells exposed to nicotinamide. Peripheral blood NK cells were prepared by T-cell (CD3+ or CD3+CD19+) depletion of the mononuclear cells as detailed in FIG. 10, and expanded in the presence of indicated concentrations (NAM 2.5 and NAM 5mM) of nicotinamide, or cytokines only (cytokines). After 2 weeks, the cultured cells were reacted with specific antibodies for the monocyte marker CD14 (FIG. 14A) or the granulocyte marker CD15 (FIG. 14B), and then monitored by FACS. Note the dramatically enhanced reduction in CD14+ or CD15+ cells in cells cultured in the presence of nicotinamide compared to controls (cytokines only);

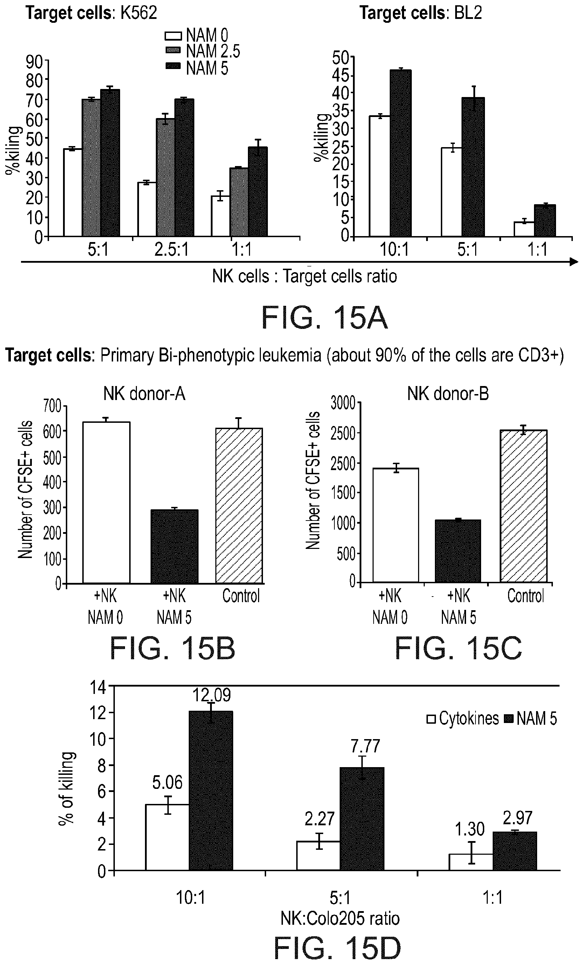

FIGs. 15A-15D are histograms illustrating the enhanced killing potential of NK cells cultured with nicotinamide of human peripheral blood NK cells exposed to nicotinamide. Peripheral blood NK cells were prepared by CD3+ or CD3+CD19+ depletion of the mononuclear cells as detailed in FIG. 10, and expanded in the presence of indicated concentrations of nicotinamide (NAM 2.5, light shading and NAM 5mM, dark shading), or cytokines only (cytokines, no shading). The peripheral blood NK cells were assayed for *ex-vivo* killing potential with K562 or BL2(15A), primary bi-phenotypic leukemia(15B and 15C) and Colo205 colon cancer (15D) target cells at E:T ratio of 1:1, 2.5:1, 5:1 or 10:1 NK cells per target cell, as indicated. Target cell death of cell lines was monitored by FACS as a percentage of dual, PI-positive and CFSE positive -labeled cells. Target cell death of primary leukemia was monitored by FACS as reduction in the percentages of CFSE labeled target cells. Enhanced target cell killing, compared to cultured controls (cytokines only, NAM 0, no shading) and fresh, non-cultured NK cells (control, hatched) strongly suggests enhanced activation of NK cells killing potential by nicotinamide;

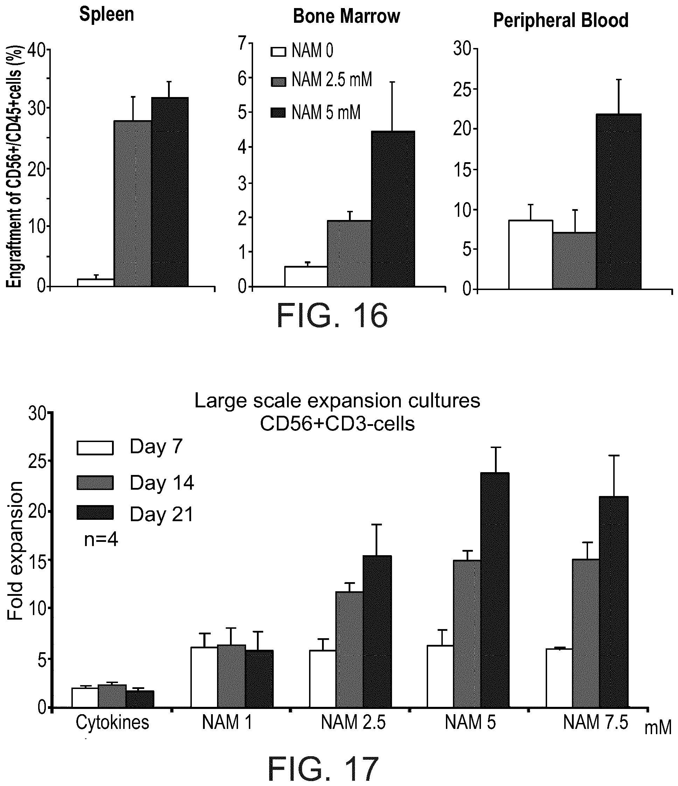

FIG. 16 is a histogram showing increased *in-vivo* functionality (homing and engraftment) of NK cells expanded in the presence of nicotinamide. Peripheral blood NK cells were prepared by CD3+ or CD3+CD19+ depletion of the mononuclear cells as detailed in FIG. 10, and expanded in the presence of indicated concentrations of nicotinamide (2.5mM NAM, light shading; 5mM NAM, dark shading), or cytokines only (NAM 0, no shading). After 2-3 weeks in culture, 15x10⁶ NK cells from each experimental group were infused into irradiated (350 Rad) NOD/SCID mice. Mice were sacrificed 4-days post infusion, and samples from spleen, bone marrow and peripheral blood were analyzed for the engraftment of human NK (CD45+CD56+) cells. Note the significantly higher *in vivo* homing/retention/engraftment of NK cells cultured with nicotinamide as compared with that of NK cells cultured without nicotinamide;
FIG. 17 is a histogram showing the dose-dependent proliferation of cord-blood derived, CD56+-purified NK cell fraction when cultured in the absence (cytokines) or presence of increasing concentrations (NAM, 1 to 7.5 mM) of nicotinamide. Cultures were initiated with cord blood NK cells purified on immunomagnetic beads for CD56+ phenotype, and maintained with cytokines (including Flt-3, IL-2 and IL-15) for up to 3 weeks. FIG. 17 shows the proliferation (fold increase, relative to day 0, initiation of the cultures) of the CD56+ NK cells at 7(no shading), 14 (light shading) and 21 (dark shading) days in culture. Note the dramatic dose-dependent increase in the expansion of the nicotinamide treated cultures, continuing throughout the entire culture period, compared to controls (cytokines);

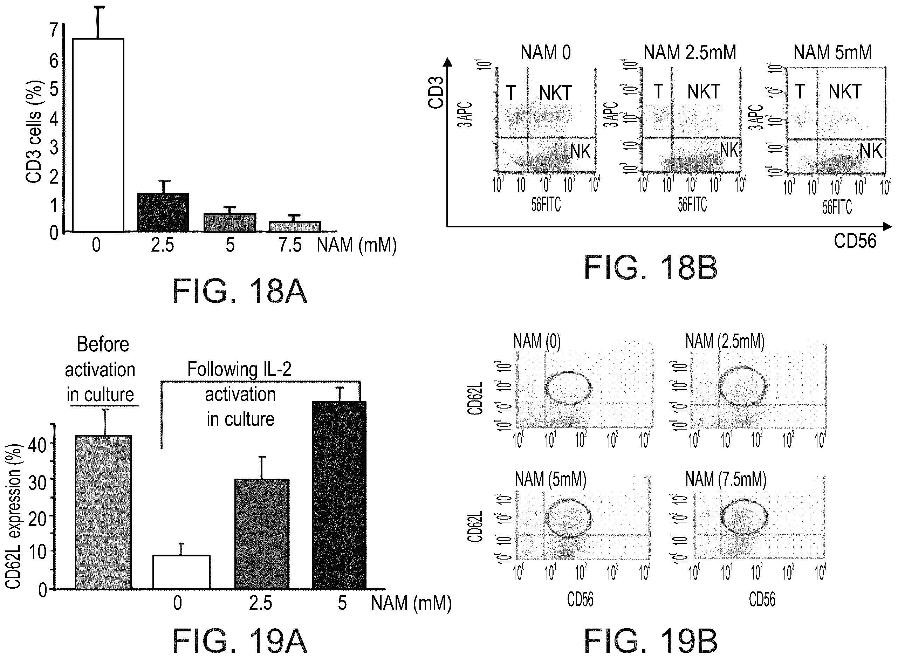

FIGs. 18A and 18B show the inhibition of T(CD3+) and NKT(CD3+CD56+) cell proliferation in cultures initiated with partially CD3-depleted peripheral blood and maintained in the absence (cytokines, 0 NAM, no shading) or presence of nicotinamide. Cultures were maintained with cytokines (including Flt-3, IL-15 and IL-2), with or without increasing concentrations (2.5, dark shading; 5mM, light shading and 7.5 mM, very light shading) of nicotinamide for 3 weeks, reacted with specific antibodies for surface markers (56FITC and 3APC), and analyzed by FACS (18B) for specific phenotypes. Note the dramatic reduction of CD3+ T cells and CD3+CD56+ NKT cells in NK cultures exposed to nicotinamide;
FIGs. 19A and 19B show the enhancement of CD62L trafficking receptor expression with nicotinamide. FIG. 19A is a histogram showing CD62L trafficking receptor expression levels on CD56+ cells purified from peripheral blood before (very light shading), and after activation in culture with IL-2, exposed for 3 weeks culture in 2.5 (light shading) and 5 (dark shading) mM nicotinamide, compared to cytokines-only controls (0 "NAM", no shading). FIG. 19B is a FACS analysis of the CD62L expression, on purified peripheral blood CD56+ cells, cultured in (2.5 - 7.5 mM) nicotinamide, or controls (NAM 0), for 3 weeks. Note the dramatically enhanced expression of CD62L in cells cultured in the presence of nicotinamide compared to controls (cytokines only);

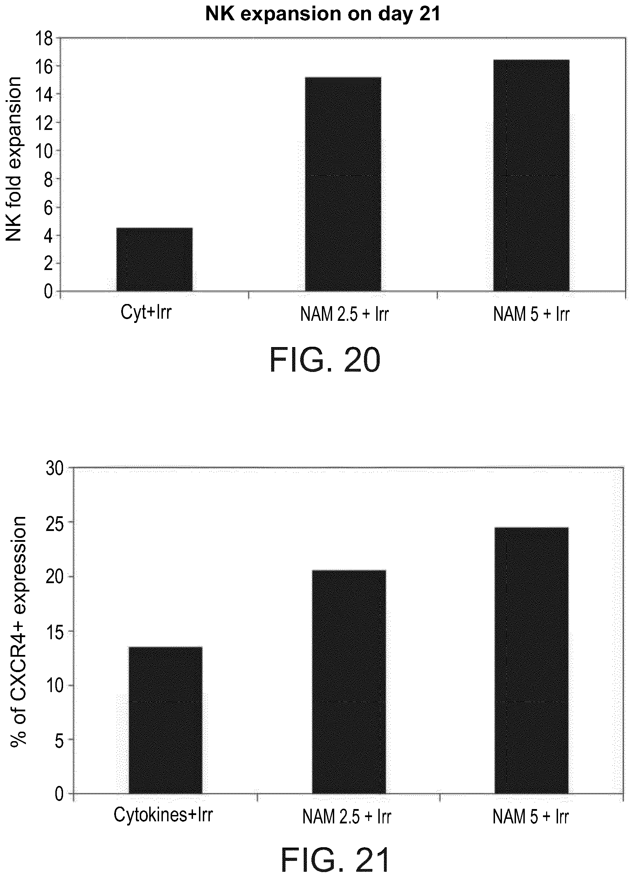

FIG. 20 is a histogram showing the effect of nicotinamide on proliferation (fold increase) of CD56+ cells purified from peripheral blood-derived mononuclear cell fractions by CD3 depletion followed by CD56+ cell selection. The purified CD56+ cells were seeded in culture (IL-2, IL-15 and Flt-3 with or without nicotinamide 2.5 and 5 mM) and with irradiated stroma derived from peripheral blood mononuclear cells from the same blood unit (Cyt+Irr, NAM 2.5+Irr and NAM 5+Irr). The ratio between the irradiated peripheral blood cells and the CD56+ selected cells was 10:1. Note the dramatically enhanced in proliferation in treated with nicotinamide compared to controls (cytokines only);
FIG. 21 is a histogram showing the effect of nicotinamide on the expression of CXCR4 on CD56+ cells in cultures treated with cytokines and irradiated stroma cells, as described in FIG. 20;

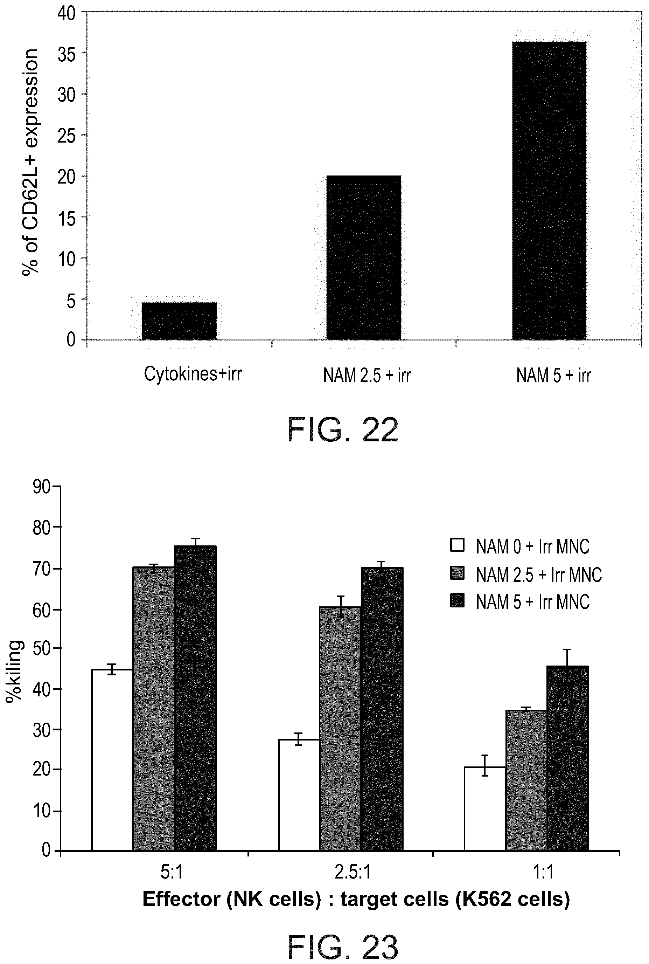

FIG. 22 is a histogram showing the effect of culture with nicotinamide on the expression of CD62L on CD56+ cells in cultures treated with cytokines and irradiated stroma cells, as described in FIG. 20;
FIG. 23 is a histogram showing the effect of culture with nicotinamide on CD56+ cells *ex-vivo* killing potential of K562 target cells, at E:T ratios of 1:1, 2.5:1 and 5:1 CD56+ cells per target cell. Target cell death was monitored by FACS as a percentage of PI-positive CFSE-labeled K562 cells. Enhanced target cell killing, with cells cultured in the indicated concentrations of nicotinamide (NAM 2.5+Irr, light shading; and NAM 5+Irr, dark shading), compared to control (cytokines only, NAM 0+Irr, no shading) strongly suggests enhanced activation of NK cells killing potential by nicotinamide, unrelated to the effects of the irradiated cells.

### DESCRIPTION OF EMBODIMENTS OF THE INVENTION

The present invention is of methods of propagating a population of natural killer (NK) cells, while at the same time, maintaining or enhancing function of the cells *ex-vivo* and/or *in-vivo.* In one embodiment, *ex-vivo* culture of NK cells with a nicotinamide and NK cell growth factors facilitates the production of NK cell populations for use as a therapeutic *ex-vivo* cultured cell preparation, which includes a propagated population of functional NK cells, in which proliferation of CD3+ cells is inhibited while NK cell proliferation is preferentially enhanced. Specifically in this respect, the present invention can be used to provide robust populations of functional NK cells, which can be used for applications in cell transplants for treatment of cancer and other disease, and in generation of NK cells suitable for genetic manipulations, which may be used for cellular gene therapy. Additional, non-limiting applications may include treatment of graft versus host disease (e.g., in bone marrow reconstitution), allogeneic and autologous adoptive immunotherapy, treatment of autoimmune disease, combination therapy with sensitizing agents and gene transfer in NK cells. The present disclosure further relates to NK cell preparations useful for transfusion and to articles-of-manufacture for preparing same.

The principles and operation of the present invention may be better understood with reference to the Examples and accompanying descriptions.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways.

Natural killer (hereinafter also abbreviated as "NK") cells are lymphoid cells that participate in immune reactions. These cells have variety of functions, especially the killing of tumor cells, cells undergoing oncogenic transformation and other abnormal cells in a living body, and are important components of innate immunological surveillance mechanisms. NK cells exhibit spontaneous non-MHC-restricted cytotoxic activity against virally infected and tumor cells, and mediate resistance to viral infections and cancer development in vivo. Thus, methods for effectively increasing the number of NK cells can be useful for treatment of tumors and elimination of virus-infected cells considered potential sources of tumor generation.

Thus, developing clinical-grade protocols (e.g., no stromal layer, minimal cytokines) for effectively expanding the number of viable NK cells and effectively enhancing their function and likelihood of homing to lymph nodes and their homeostatic proliferation *in-vivo* following infusion, could improve the success of adoptive immunotherapy with NK cells for the treatment of solid tumors, hematopoietic malignancies, viral and autoimmune disorders and the like.

The present invention is based on the discovery that *ex-vivo* exposure of NK cells to nicotinamide above a certain concentration, as is further detailed herein, during culture effectively enhances proliferation and/or functionality of functionally competent NK cells, and results in significant reduction in the T cell fraction of the culture. As such, in an embodiment thereof, the present invention provides clinically appropriate culture conditions capable of efficiently inducing the proliferation and/or function of functionally mature NK cells *ex-vivo* and *in-vitro,* without concomitant induction of non-NK cell (e.g.CD3+) proliferation.

Thus, according to one aspect of an embodiment of the present invention there is provided a method of *ex-vivo* culturing natural killer (NK) cells, the method comprising culturing a population of cells comprising NK cells with at least one growth factor and an effective concentration, effective exposure time and effective duration of exposure of nicotinamide, wherein culturing the NK cells with the at least one growth factor and the effective concentration, effective exposure time and effective duration of the nicotinamide results in at least one of the following:
(a) elevated expression of CD62L as compared to NK cells cultured under otherwise identical culturing conditions with less than 0.1 mM of the nicotinamide; and
(b) increased cytotoxic activity as compared to NK cells cultured under otherwise identical culturing conditions with less than 0.1 mM of the nicotinamide.

As used herein, the term natural killer (NK) cells refers to large granular lymphocytes involved in the innate immune response. Functionally, NK cells exhibit cytolytic activity against a variety of targets via exocytosis of cytoplasmic granules containing a variety of proteins, including perforin, and granzyme proteases. Killing is triggered in a contact-dependent, non-phagocytotic process which does not require prior sensitization to an antigen. Human NK cells are characterized by the presence of the cell-surface markers CD16 and CD56, and the absence of the T cell receptor (CD3). Human bone marrow-derived NK cells are further characterized by the CD2+CD16+CD56+CD3- phenotype, further containing the T-cell receptor zeta-chain [zeta(ζ)-TCR], and often characterized by NKp46, NKp30 or NKp44. Non- NK cells such as NKT cells or CD8NKT possess characteristics and cell-surface markers of both T cells and NK cells. In one embodiment, the method of the present invention is employed for *ex-vivo* propagation of mature NK cells from a population of cells. As used herein, the term "mature NK cell" is defined as a committed NK cell, having characteristic surface markers and NK cell function, and lacking the potential for further differentiation. As use herein, mature NK cells include, but are not limited to CD56^{bright} cells, which can proliferate and produce abundant cytokines, CD56^{dim} cells, exhibiting robust cytotoxicity, CD56^{bright}CD94^{high} and CD56^{dim}CD94^{high} cells. In another embodiment, NK progenitor cells, or mixed populations of NK progenitor cells and mature NK cells are propagated. Cell surface expression of the CD56, CD3, CD16, CD94 and other markers can be determined, for example, via FACS analysis or immunohistological staining techniques.

As used herein, the term "progenitor" refers to an immature cell capable of dividing and/or undergoing differentiation into one or more mature effector cells. Lymphocyte progenitors include, for example, pluripotent hematopoietic stem cells capable of giving rise to mature cells of the B cell, T cell and NK lineages. In the B cell lineage (that is, in the developmental pathway that gives rise to mature B cells), progenitor cells also include pro-B cells and pre-B cells characterized by immunoglobulin gene rearrangement and expression. In the T and NK cell lineages, progenitor cells also include bone-marrow derived bipotential T/NK cell progenitors [e.g., CD34(+)CD45RA(hi)CD7(+) and CD34(+)CD45RA(hi)Lin(-)CD10(+) cells], as well as intrathymic progenitor cells, including double negative (with respect to CD4 and CD8) and double positive thymocytes (T cell lineage) and committed NK cell progenitors. Hematopoietic progenitors include CD34+ and early progenitors such as CD133+, CD34+CD38- and CD34+Lin- cells.

As used herein the term *"ex-vivo"* refers to a process in which cells are removed from a living organism and are propagated outside the organism (e.g., in a test tube). As used herein, the term *"in-vitro"* refers to a process by which cells known to propagate only *in-vitro,* such as various cell lines are cultured.

*Ex-vivo* expansion of NK cells can be effected, according to this aspect of the present invention, by providing NK cells *ex vivo* with conditions for cell proliferation and *ex vivo* culturing the NK cells with a nicotinamide moiety, thereby *ex-vivo* propagating the population of NK cells.

As used herein "culturing" includes providing the chemical and physical conditions (e.g., temperature, gas) which are required for NK cell maintenance, and growth factors. In one embodiment, culturing the NK cells includes providing the NK cells with conditions for proliferation. Examples of chemical conditions which may support NK cell proliferation include but are not limited to buffers, nutrients, serum, vitamins and antibiotics as well as cytokines and other growth factors which are typically provided in the growth (i.e., culture) medium. In one embodiment, the NK culture medium includes MEMα comprising 10 % FCS or CellGro SCGM (Cell Genix) comprising 5 % Human Serum/LiforCell® FBS Replacement (Lifeblood Products). Other media suitable for use with the invention include, but are not limited to Glascow's medium (Gibco Carlsbad CA), RPMI medium (Sigma-Aldrich, St Louis MO) or DMEM (Sigma-Aldrich, St Louis MO). It will be noted that many of the culture media contain nicotinamide as a vitamin supplement for example, MEMα (8.19 µM nicotinamide), RPMI (8.19 µM nicotinamide), DMEM (32.78 µM nicotinamide) and Glascow's medium (16.39 µM nicotinamide), however, the methods of the present invention relate to exogenously added nicotinamide supplementing any nicotinamide included the medium's formula, or that resulting from overall adjustment of medium component concentrations.

According to some embodiments of the present invention, culturing the NK cells with growth factors comprises providing the cells with nutrients and with at least one growth factor, wherein said at least one growth factor is interleukin-15 (IL-15. The use of other cytokines and growth factors is contemplated, for example, addition of IL-1, TNF-α, etc. Cytokines and other growth factors are typically provided in concentrations ranging from 0.5-100ng/ml, or 1.0-80ng/ml, more typically 5-750ng/ml, yet more typically 5.0-50ng/ml (up to 10X such concentrations may be contemplated), and are available commercially, for example, from Perpo Tech, Inc., Rocky Hill, NJ, USA. In one embodiment, the at least one growth factor is IL-2. In another embodiment, the growth factor is IL-15. In yet another embodiment, NK cells are cultured with IL-2 and IL-15 .

Further, it will be appreciated in this respect that novel cytokines are continuously discovered, some of which may find uses in the methods of NK cell proliferation of the present invention. For applications, in which cells are introduced (or reintroduced) into a human subject, it is often preferable to use serum-free formulations, such as AIM V.^{RTM} serum free medium for lymphocyte culture or MARROWMAX.^{RTM} bone marrow medium. Such medium formulations and supplements are available from commercial sources such as Invitrogen (GIBCO) (Carlsbad, Calif). The cultures can be supplemented with amino acids, antibiotics, and/or with cytokines to promote optimal viability, proliferation, functionality and/or and survival.

According to one embodiment, the cells are cultured with growth factors and nicotinamide. As used herein, the term "nicotinamide moiety" refers to nicotinamide. Nicotinamide derivatives, analogs and metabolites can be screened and evaluated for their effect on *ex-vivo* NK proliferation in culture by addition to NK cultures maintained as described hereinbelow, addition to functional assays such as killing and motility assays (see Examples section), or in automated screening protocols designed for high-throughput assays well known in the art.

As used herein, the phrase "nicotinamide analog" refers to any molecule that is known to act similarly to nicotinamide in the abovementioned or similar assays. Representative examples of nicotinamide analogs can include, without limitation, benzamide, nicotinethioamide (the thiol analog of nicotinamide), nicotinic acid and α-amino-3-indolepropionic acid.

The phrase "nicotinamide derivative" further refers to any structural derivative of nicotinamide itself or of an analog of nicotinamide. Examples of such derivatives include, without limitation, substituted benzamides, substituted nicotinamides and nicotinethioamides and N-substituted nicotinamides and nicotinthioamides, 3-acetylpiridine and sodium nicotinate

The nicotinamide moiety of the invention is nicotinamide.

As used herein, the phrase "effective concentration" of nicotinamide is defined as that concentration of nicotinamide which, when provided to the population of NK cells in culture, for an effective duration of exposure to the nicotinamide and at an effective time of exposure to nicotinamide in culture, results in one or more of elevated expression of CD62L, elevated migration response, elevated homing and in-vivo retention, greater proliferation and increased cytotoxic activity of the NK cells, as compared to NK cells cultured under identical conditions but with less than 0.1 mM of the nicotinamide. Nicotinamide concentrations suitable for use in some embodiments disclosed herein are typically in the range of about 0.5 mM to about 50 mM, about 1.0 mM to about 25 mM, about 1.0 mM to about 25 mM, about 2.5 mM to about 10 mM, about 5.0 mM to about 10 mM. Examples I-VII below demonstrate exemplary effective concentrations of the nicotinamide moiety (nicotinamide) of about 0.5 to about 10 mM, typically 2.5 or 5.0 mM, based on the effect of these concentrations of nicotinamide on proliferation and NK cell function. According to some embodiments disclosed herein, nicotinamide concentrations in the range (mM) of about 0.5, about 0.75, about 1.0, about 1.25, about 1.5, about 1.75, about 2.0, about 2.25, about 2.5, about 2.75, about 3.0, about 3.25, about 3.5, about 3.75, about 4.0, about 4.25, about 4.5, about 4.75, about 5.0, about 5.25, about 5.5, about 5.75, about 6.0, about 6.25, about 6.5, about 6.75, about 7.0, about 7.25, about 7.5, about 7.75, about 8.0, about 8.25, about 8.5, about 8.75, about 9.0, about 9.25, about 9.5, about 9.75, about 10.0, about 11.0, about 12.0, about 13.0, about 14.0, about 15.0, about 16.0, about 17.0, about 18.0 and about 20.0 mM, and all effective intermediate concentrations are contemplated.

Effective concentrations of the nicotinamide can be determined according to any assay of NK proliferation and/or activity, for example, cell culture or function protocols as detailed in Examples I-VI below. According to one embodiment, an effective concentration of nicotinamide is a concentration which use thereof in culture "enhances", or results in a net increase of proliferation and/or function of NK cells in culture, compared to "control" cultures having less than 0.1 mM of the nicotinamide and tested from the same cord blood, bone marrow or peripheral blood preparation, in the same assay and under similar culture conditions (duration of exposure to nicotinamide , time of exposure to nicotinamide).

As used herein, the phrase "effective duration of time" of exposure of the NK cells to nicotinamide is defined as that duration of exposure to nicotinamide during which, when the nicotinamide and/or other nicotinamide is provided to the population of NK cells in culture in an effective concentration and at an effective time of exposure, results in one or more of elevated expression of CD62L, elevated migration response, elevated homing and in-vivo retention, greater proliferation and increased cytotoxic activity of the NK cells, as compared to NK cells cultured under identical conditions without with less than 0.1 mM nicotinamide. Duration of exposure of the NK cell populations to nicotinamide suitable for use in some embodiments disclosed herein are typically in the range of about 2 hours to about 5 weeks, about 30 hours to about 4 weeks, about 2 days to about 3 weeks, about 1 week, about 2 weeks, about 3 weeks. Examples I-VI below demonstrate exemplary effective durations of exposure to nicotinamide of about 1 week to about 3 weeks, based on the effect of the exposure to nicotinamide on proliferation and NK cell function. According to some embodiments disclosed herein, duration of exposure to nicotinamide is about 1.0, about, about 2.0, about 2.5, about 3.0, about 3.5, about 4.0, about 4.5, about 5.0, about 6.0, about 7.0, about 8.0, about 9.0, about 10.0, about 11.0, about 12.0, about 13.0, about 14.0, about 15.0, about 16.0, about 17.0, about 18.0, about 19.0, about 20.0, about 21.0 days, about 25 days, about 30 days, about 35 days and all effective intermediate durations are contemplated. Effective durations of time of exposure to nicotinamide can be determined according to any assay of NK proliferation and/or activity, for example, cell culture or function protocols as detailed in Examples I-VI below.

It will be appreciated that exposure of the NK cell populations to nicotinamide can be initiated with establishment of the cell culture, or at any time during cell culture, even for a short duration just prior to use (e.g., infusion) of NK cells. As used herein, the phrase "effective exposure time" of the NK cell population to nicotinamide is defined as the time at which, during the culture of the NK population, the nicotinamide is provided to the population of NK cells, in an effective concentration of the nicotinamide and for an effective duration of time, resulting in one or more of elevated expression of CD62L, and increased cytotoxic activity of the NK cells, as compared to NK cells cultured under identical conditions without with less than 0.1 mM of the nicotinamide. Time of exposure of the NK cell populations to nicotinamide suitable for some embodiments of the present invention is typically from seeding of the NK cells to about 5 weeks after culturing, from about 1 hour after seeding to about 3 weeks after culturing, from about 24 hours to about 3 weeks after culturing, and from the time of seeding of the NK cell population in culture. According to some embodiments of the invention, time of exposure of the NK cells to the nicotinamide is at seeding, about 2 hours after seeding of the cells, about 12 hours after seeding of the cells, about 24 hours after seeding of the cells, about 2 days after seeding of the cells, about 4 days after seeding of the cells, about 7 days after seeding of the cells, about 8.0, about 9.0, about 10.0, about 11.0, about 12.0, about 13.0, about 14.0, about 15.0, about 16.0, about 17.0, about 18.0, about 19.0, about 20.0, about 21.0 days, about 25 days, about 30 days, about 35 days after seeding of the cells and all effective intermediate times are contemplated. Effective times of exposure to the nicotinamide can be determined according to any assay of NK proliferation and/or activity, for example, cell culture or function protocols as detailed herein, for example, in Examples I-VI hereinbelow.

As detailed in the Examples section that follows, culturing NK cell populations with at least one growth factor and effective concentrations of nicotinamide, provided at an effective exposure time for an effective duration of culture, results in at least one of enhanced proliferation and/or enhanced NK cell function of the cultured cells, as compared to NK cells cultured under identical conditions in less than 0.1 mM nicotinamide.

As used herein, the term "propagation" or "proliferation" refers to growth, for example, cell growth, and multiplication of cell numbers. Propagation and proliferation, as used herein relate to increased numbers of NK cells accruing during the incubation period. Propagation in vitro or in vivo of cells displaying the phenotype of NK cells is a known phenomenon following their stimulation, for example with IL-2, Epstein-Barr virus-transformed lymphoblastoid lines and others.

Assays for cell proliferation well known in the art, including, but not limited to clonogenic assays, in which cells are seeded and grown in low densities, and colonies counted, mechanical assays [flow cytometry (e.g., FACS™), propidium iodide], which mechanically measure the number of cells, metabolic assays (such as incorporation of tetrazolium salts e.g., XTT, MTT, etc), which measure numbers of viable cells, direct proliferation assays (such as BUdR, thymidine incorporation, and the like), which measure DNA synthesis of growing populations. In one embodiment, cell proliferation of populations of NK cells cultured with an effective concentrations of nicotinamide according to the present invention is measured at a predetermined time after seeding NK cells in culture (for example, about 10 hours, 12 hours, about 1, 2, 3, 4, 5, 6, 7 days, about 1, 2, 3, 4, 5 weeks, 2 months or more) is determined by FACS analysis, using anti-CD56 and anti-CD3 markers to identify and quantitate the CD56+CD3- NK cell fraction of the population. Proliferation of NK cells can be expressed as the fold increase, (e.g., expansion or fold expansion) of NK cells, as compared to the original NK cell fraction before culture. In some embodiments, populations of NK cells exposed to effective concentrations of nicotinamide according to the present invention have a fold increase of the NK cell population of at least 2X, at least 10X, at least 20X, at least 40X, at least 50X, at least 75X, at least 100X, at least 150X, at least 250X and at least 500X or more, after about 5, about 7, about 12, about 14, about 18, about 21, about 25, about 30 or more days culture. In another embodiment, the fold expansion of populations of NK cells, as determined by FACS™, exposed to effective concentrations of nicotinamide is at least about 1.2X, about 1.3X, about 1.5X, about 1.75X, about 2X, about 2.25X , about 2.5X, about 2.75X , about 3.0, about 3.5X, about 4X, about 4.5X, about 5X, about 6X, about 7X, about 8X, about 9X, about 10X, more than that of NK cells cultured in identical conditions with less than 0.1mM nicotinamide.

As used herein, the term "function" or "NK cell function" refers to any biological function ascribed to NK cells. A non-limiting list of NK cell functions includes, for example, cytotoxicity, induction of apoptosis, cell motility, directed migration, cytokine and other cell signal response, cytokine/chemokine production and secretion, expression of activating and inhibitory cell surface molecules *in-vitro,* cell homing and engraftment (in-vivo retention) in a transplanted host, and alteration of disease or disease processes *in vivo.* In some embodiments, NK cell functions enhanced by exposure to nicotinamide include at least one of elevated expression of CD62L surface marker, elevated migration response, and greater cytotoxic activity of the NK cells, as well as elevated homing and in-vivo retention of infused NK cells.

Assays for adhesion and migration molecules such as CD62L, CXCR-4, CD49e and the like, important for homing/engraftment and retention of cells in transplantation, are well known in the art. CD62L expression in a cell can be assayed, for example, by flow cytometry, immunodetection, quantitative cDNA amplification, hybridization and the like. In one embodiment, CD62L expression is detected in different populations of NK cells by exposure of the cells to a fluorescent-tagged specific anti-human CD62L monoclonal antibody [e.g., CD62L PE, Cat. No. 304806 from BioLegend (San Diego, CA, USA)], and sorting of the cells by fluorescent activated cell sorting (FACS). In some embodiments, populations of NK cells exposed to effective concentrations of nicotinamide have at least 25 %, at least 30 %, at least 40 % or more of the cells detected expressing CD62L, as determined by FACS™. In another embodiment, populations of NK cells exposed to nicotinamide according to the present invention have at least about 1.2X, about 1.3X, about 1.5X, about 1.75X, about 2X, about 2.25X , about 2.5X, about 2.75X , about 3.0, about 3.5X, about 4X, about 4.5X, about 5X, about 6X, about 7X, about 8X, about 9X, about 10X or more expression of CD62L, as determined by FACS™, when compared to NK cells cultured in identical conditions with less than 0.1mM of the nicotinamide.

Assays for cells migration are well known in the art. Migration of cells can be assayed, for example, by transmigration assays or gap closure assays. In transmigration assays, such as the two-chamber technique, cells are separated from a stimulus by a barrier (e.g., filter), and migration of the cells is detected by counting loss of cells from the origin, accumulation of cells across the barrier, or both, at specific intervals. In the gap closure assay, cells are placed on the periphery of a visible gap (scored agar plate, around a circle, etc) and incubated with a stimulus. Closure of the space between the cells applied by cell motility, in response to a stimulus, is visualized using cytometry, immunodetection, microscopy/morphometrics, etc. In one embodiment, migration potential of different populations of NK cells is determined by the "Transwell"™ transmigration assay, in response to SDF (250 ng/ml). In some embodiments, populations of NK cells exposed to effective concentrations of the nicotinamide according to the present invention have least 40 %, at least 50 %, at least 60 %, at least 70 % and at least 80 % or more migration measure by the Transwell assay described herein. In another embodiment, populations of NK cells exposed to effective concentrations of the nicotinamide have at least about 1.2X, about 1.3X, about 1.5X, about 1.75X, about 2X, about 2.25X , about 2.5X , about 2.75X , about 3.0, about 3.5X, about 4X, about 4.5X, about 5X, about 6X, about 7X, about 8X, about 9X, about 10X or more migration, as determined by the transwell assay, when compared to NK cells cultured in identical conditions with less than 0.1 mM of the nicotinamide.

Assays for homing and in-vivo retention of transfused or transplanted cells are well known in the art. As used herein, the term "homing" refers to the ability of a transfused or transplanted cell to reach, and survive, in a host target organ. For example, NK cells target organs can be the lymphoid tissue, hepatocytes target organs can be liver parenchyma, alveolar cells target organs can be lung parenchyma, etc. As used herein, the term "in-vivo retention" (also known as "engraftment") refers to the ability of the transfused or transplanted cells to proliferate and remain viable in the target organs. Animal models for assaying homing and in-vivo retention of transplanted NK cells include, but are not limited to immunodeficient small mammals (such as SCID and IL2Rγ^{null} mice and the like). The SCID-Hu mouse model employs C.B-17 scid/scid (SCID) mice transplanted with human fetal thymus and liver tissue or fetal BM tissue and provides an appropriate model for the evaluation of transplanted human NK cells retention and therapeutic potential. Homing and in-vivo retention of transplanted cells can be assessed in human host subjects as well. In one embodiment, homing and in-vivo retention is assayed in irradiated NOD/SCID mice (see Example VI herein), transfused with, for example, about 15X10⁴, about 15X10⁵, about 15X10⁶, about 15X10⁷ or more human NK cells cultured with an effective concentrations of nicotinamide according to the present invention, and sacrificed at a predetermined time post transfusion (for example, about 5 hours, 10 hours, 12 hours, 1, 2, 3, 4, 5, 6, 7 days, 1, 2, 3, 4, 5 weeks, 2, 3, 4 months or more post transfusion). Upon sacrifice of the mice, samples of spleen, bone marrow, peripheral blood, and other organs are evaluated by FACS for the presence of human NK cells (CD56+CD45+) using human specific Abs. Percent in vivo retention is expressed as the percent of cells of the organ displaying the donor phenotype (e.g., CD45 for human cells). In some embodiments, target organs (e.g., lymphoid tissue such as bone marrow, spleen, thymus, lymph nodes, GALT) of host mice transfused with populations of NK cells exposed to effective concentrations of nicotinamide according to the present invention have at least 5 %, at least 10 %, at least 20 %, at least 25 %, at least 30 %, at least 40 %, at least 50 %, at least 60 %, at least 70 % and at least 80 % or more homing and in vivo retention. In one specific embodiment, 15X10⁶ NK cells cultured with an effective concentration of nicotinamide according to the present invention are transfused to irradiated SCID mice, and at least 25 % NK cells having donor-specific lineage (e.g., CD45+) are detected in the host spleen, 4 days after the transfusion. In another embodiment, populations of NK cells exposed to effective concentrations of nicotinamide have at least about 1.2X, about 1.3X, about 1.5X, about 1.75X, about 2X, about 2.25X , about 2.5X , about 2.75X , about 3.0, about 3.5X, about 4X, about 4.5X, about 5X, about 6X, about 7X, about 8X, about 9X, about 10X or more homing and in-vivo retention, as determined by FACS™, when compared to homing and in-vivo retention of NK cells cultured in identical conditions with less than 0.1mM nicotinamide.

As used herein, the term "homeostatic proliferation" refers to proliferation within the target organ or tissue capable of maintaining stable numbers of the infused NK cells over time, preferably months or years.

Currently many clinical trials involving transplantation of NK cells into patients are being conducted, for conditions including, for example, but not exclusively, leukemia (NCT 00799799 and NCT 00303667), hematological malignancies (NCT 00697671, NCT 00354172 and 00640796), post-ASCT (NCT 00586703), neuroblastoma (NCT 00698009), malignant melanoma (NCT 00846833), combination therapy with chemotherapy (NCT 00625729), solid tumors (NCT 00640796) and nasopharyngeal carcinoma (NCT 00717184) and for diverse malignancies (NCT01105650). A complete, current and detailed list of current clinical trials and detailed protocols for NK cell therapy is available at the U.S. National Institutes of Health Clinical Trial website.

Assays for cytotoxicity ("cell killing") are well known in the art. Examples of suitable target cells for use in redirected killing assays are cancer cell line, primary cancer cells solid tumor cells, leukaemic cells, or virally infected cells. Particularly, K562, BL-2, colo250 and primary leukaemic cells can be used, but any of a number of other cell types can be used and are well known in the art (see, e.g., Sivori et al. (1997) J. Exp. Med. 186: 1129-1136; Vitale et al. (1998) J. Exp. Med. 187: 2065-2072; Pessino et al. (1998) J. Exp. Med. 188: 953-960; Neri et al. (2001) Clin. Diag. Lab. Immun. 8:1131-1135). Cell killing is assessed by cell viability assays (e.g., dye exclusion, chromium release, CFSE), metabolic assays (e.g., tetrazolium salts), and direct observation. In one embodiment, cytotoxicity potential of different populations of NK cells is determined by CFSE retention and PI uptake in cells exposed to NK cells at E:T of 1:1, 2.5:1, 5:1, or 10:1, and populations of NK cells exposed to effective concentrations of nicotinamide according to the present invention kill at least 20 %, at least 25 %, at least 30 %, at least 40 %, at least 50 %, at least 60 %, at least 70 % and at least 80 % or more of the target cells, as measured by the dye exclusion assay described herein. In another embodiment, populations of NK cells exposed to effective concentrations of nicotinamide have at least about 1.2X, about 1.3X, about 1.5X, about 1.75X, about 2X, about 2.25X , about 2.5X , about 2.75X , about 3.0, about 3.5X, about 4X, about 4.5X, about 5X, about 6X, about 7X, about 8X, about 9X, about 10X or more killing potential, as determined by the dye exclusion assay, when compared to NK cells cultured in identical conditions with less than 0.1 %mM of the nicotinamide.

Culturing the NK cells can be effected with or without feeder cells or a feeder cell layer. Feeder layer-free *ex-vivo* culture is highly advantageous for clinical applications of cultured cells, including NK cells. As detailed in the Examples section below, effective enhancement of NK cell *ex-vivo* proliferation and cell function was observed in feeder layer and feeder cell-free long and short term NK cell cultures derived from selected and unselected cord blood and bone marrow cells. Thus, according to one embodiment, culturing the population of NK cells is effected without feeder layer or feeder cells.

According to some embodiments of the present invention, and as detailed in the Examples section which follows, the NK cell population is cultured with IL-2 and 5 mM nicotinamide, the exposure time to nicotinamide is from seeding of the population of cells comprising NK cells, and the exposure duration is from about 2 weeks to about 3 weeks, optionally 2 weeks, and optionally 3 weeks.

In some embodiments of the present invention, populations of NK cells exposed to effective concentrations of nicotinamide according to the present invention can have at least any two, optionally any three, optionally any four and optionally all five of elevated expression of CD62L surface marker, elevated migration response, and greater cytotoxic activity of the NK cells, as well as elevated homing and in-vivo retention of infused NK cells, as compared to NK cells cultured in identical conditions with less than 0.1 mM of the nicotinamide. In one particular embodiment, populations of NK cells exposed to effective concentrations of nicotinamide according to the present invention have greater proliferation, elevated CD62L expression, and elevated homing and in-vivo retention of infused NK cells, as compared to NK cells cultured in identical conditions with less than 0.1 mM of the nicotinamide.

As detailed herein, enhancement of NK cell proliferation and cellular function by exposure to nicotinamide is observed as well in the presence of feeder cells. Thus, in another embodiment, the NK cells are cultured in the presence of feeder cells or a feeder layer. Typically, feeder layers comprise irradiated stromal cells, cells of immortalized cell lines, and the like. Methods for culturing NK cells on feeder layers or with feeder cells are described in detail in, for example, Frias et al. (Exp Hematol 2008; 36: 61-68), Harada et al. (Exp Hematol 2004;32:614-21), Campana et al. (US20090011498) Childs et al. (US20090104170) and Tsai (US20070048290).

NK cells of the present invention may be derived from any source which comprises such cells. NK cells are found in many tissues, and can be obtained, for example, from lymph nodes, spleen, liver, lungs, intestines, deciduas and can also be obtained from iPS cells or embryonic stem cells (ESC). Typically, cord blood, peripheral blood, mobilized peripheral blood and bone marrow, which contain heterogeneous lymphocyte cell populations, are used to provide large numbers of NK cells for research and clinical use. Thus, according to one aspect of one embodiment of the present invention, the method comprises culturing a population of NK cells derived from one of cord blood, peripheral blood or bone marrow. As detailed herein, it was uncovered that significant differences in the proportions of lymphocyte cell types are found between NK cell preparations from different sources. For example, CD56+ cells isolated (by immunomagnetic isolation) from cord blood typically include a greater proportion of CD56+CD3- NK cells and fewer NKT cells co- expressing CD56 NK marker and CD3 T cell marker (CD56+CD3+) than the CD56+ fraction of bone marrow or peripheral blood (see Examples I-VI herein). Thus, in certain embodiments, NK cells are cultured from a heterogeneous cell population comprising NK cells, CD3- cells and CD3+ cells. In one embodiment the CD3+ fraction is greater than the CD3- NK cell fraction, as is typical of bone marrow, cord blood or peripheral blood. In yet another embodiment, the NK cell population is selected or enriched for NK cells. In some embodiments NK cells can be propagated from fresh cell populations, while other embodiments propagate NK cells from stored cell populations (such as cyropreserved and thawed cells) or previously cultured cell populations.

NK cells are associated with mononuclear cell fraction of cord blood or peripheral blood or bone marrow. In one embodiment, the population of cells comprising said NK cells is a mononuclear or total nuclear cell population depleted of CD3+ cells, or CD3+ and CD19+ cells. In another embodiment, the population of cells comprising the NK cells is an unselected NK cell population. In yet another embodiment, the cells are further selected and the NK cells comprise CD56+CD16+CD3- cells and or CD56+CD16-CD3-. Methods for selection of NK cells according to phenotype (e.g., immunodetection and FACS analysis) are detailed herein, for example, in the Methods section that follows.

Most commonly, whole blood or bone marrow samples are further processed to obtain populations of cells prior to placing the lymphocytes into culture medium (or buffer). For example, the blood or bone marrow sample can be processed to enrich or purify or isolate specific defined populations of cells. The terms "purify" and "isolate" do not require absolute purity; rather, these are intended as relative terms. Thus, for example, a purified lymphocyte population is one in which the specified cells are more enriched than such cells are in its source tissue. A preparation of substantially pure lymphocytes can be enriched such that the desired cells represent at least 50 % of the total cells present in the preparation. In certain embodiments, a substantially pure population of cells represents at least 60 %, at least 70 %, at least 80 %, at least 85 %, at least 90 %, or at least 95 % or more of the total cells in the preparation.

Methods for enriching and isolating lymphocytes are well known in the art, and appropriate methods can be selected based on the desired population. For example, in one approach, the source material is enriched for lymphocytes by removing red blood cells. In its simplest form, removal of red blood cells can involve centrifugation of unclotted whole blood or bone marrow. Based on density red blood cells are separated from lymphocytes and other cells. The lymphocyte rich fractions can then be selectively recovered. Lymphocytes and their progenitors can also be enriched by centrifugation using separation mediums such as standard Lymphocyte Separation Medium (LSM) available from a variety of commercial sources. Alternatively, lymphocytes/progenitors can be enriched using various affinity based procedures. Numerous antibody mediated affinity preparation methods are known in the art such as antibody conjugated magnetic beads. Lymphocyte enrichment can also be performed using commercially available preparations for negatively selecting unwanted cells, such as FICOLL-HYPAQUE™ and other density gradient mediums formulated for the enrichment of whole lymphocytes, T cells or NK cells.

Methods of selection of NK cells from blood, bone marrow or tissue samples are well known in the art (see, for example, US Patent NO. 5,770,387 to Litwin et al). Most commonly used are protocols based on isolation and purification of CD56+ cells, usually following mononuclear cell fractionation, and depletion of non-NK cells such as CD3+, CD34+, CD133+ and the like. Combinations of two or more protocols can be employed to provide NK cell populations having greater purity from non-NK contaminants. The purity of the NK cell preparation is of great significance for clinical applications, as non-NK cells, such as T-cells and NKT cells, contribute to antigen-specific reactions such as GVHD, compromising the potential benefits of NK cell transplantation. Commercially available kits for isolation of NK cells include one-step procedures (for example, CD56 microbeads and CD56+, CD56+CD16+ isolation kits from Miltenyi Biotec, Auburn CA), and multistep procedures, including depletion, or partial depletion, of CD3+ or depletion with non-NK cell antibodies recognizing and removing T cells (for example, OKT-3), B cells, stem cells, dendritic cells, monocytes, granulocytes and erythroid cells. Thus, in some embodiments, the NK cells are selected CD56+CD3-, CD56+CD16+CD3-, CD56+CD16-CD3- or other purified NK cell populations. It will be noted, however, that clinical applications typically favor fewer manipulations of the candidate cell population.

In one embodiment, the NK cells are propagated *ex-vivo* by short or long term culture. As detailed in Example I, culture of NK cells with growth factors and nicotinamide, according to the methods of the present invention, for as little as 7 days, or as many as 3 weeks resulted in enhanced, preferential proliferation and/or functionality of the cultured NK cells, as compared to cells cultured with cytokines but with less than 0.1 mM nicotinamide. Thus, in some embodiments of the present invention, culturing the NK cell population is for at least 3, least 5, at least 7, optionally 10, optionally 12, optionally 14, optionally 16, optionally 18, optionally 20 and optionally 21 days, or 1, 2 or three weeks, four weeks, five weeks, six weeks, or more. Exemplary, non-limiting culture durations, as detailed in Examples I to VI, are 7 days (1 week) and 21 days (3 weeks).

NK cell populations can be cultured using a variety of methods and devices. Selection of culture apparatus is usually based on the scale and purpose of the culture. Scaling up of cell culture preferably involves the use of dedicated devices. Apparatus for large scale, clinical grade NK cell production is detailed, for example, in Spanholtz et al. (PLoS ONE 2010;5:e9221) and Sutlu et al. (Cytotherapy 2010, Early Online 1-12).

Peled et al. (WO 03/062369) have recently disclosed a dramatic effect of nicotinamide treatment on engraftment potential of CD34+ cells. This effect of nicotinamide has been observed both with cells proliferating in culture, and for short durations of incubation, sufficient for minimal or no cell division. Thus, while enhancing proliferation of NK cells in *ex-vivo* culture is an important goal of the present invention, short term *ex-vivo* exposure of NK cells to nicotinamide, for periods of minutes, hours, 1 day, and the like is envisaged. Such short term exposure of NK cells to nicotinamide, for periods of time not sufficient for proliferation, can potentially enhance, for example, NK cell functionality (cytotoxicity, migration potential, cell surface molecule expression, engraftment potential and the like). Short term treatment with nicotinamide can be provided to fresh cells, cryopreserved and thawed cells, cells in culture, purified cells, mixed cell populations, and the like. In one embodiment, such short term nicotinamide treatment is provided immediately before use (transplantation, infusion, etc) of the NK cells.

The inventors have surprisingly observed that culture of a mixed cell population comprising NK (CD56+) cells and non-NK cells (e.g., T (CD3+) cells, NKT (CD56+CD3+) cells and the like) in the presence of an effective concentration of nicotinamide in the culture medium not only enhances NK cell proliferation, growth and functionality, but also inhibits the proliferation and growth of the non-NK (e.g.,T and NKT cells) in the same culture (see Example V herein). Thus, in one embodiment of the invention culturing a heterogeneous population of NK and CD3+ cells with an effective concentration of nicotinamide results in a population of NK cells having a reduced ratio of CD3+ to CD56+CD3- cells, as compared to a population of NK cells cultured under otherwise identical cultural conditions with less than 0.1 mM of the nicotinamide. In yet another embodiment, culturing a heterogeneous population of NK and CD3+ cells with an effective concentration of nicotinamide, according to the method of the invention results in a population of NK cells having reduced numbers of CD14+ and CD15+ cells, as compared to a population of NK cells cultured under otherwise identical cultural conditions with less than 0.1 mM of the nicotinamide.

The methods described hereinabove for *ex-vivo* culturing NK cells populations can result, *inter alia,* in a cultured population of NK cells.

Thus, further according to an aspect of the present invention there is provided a population of NK cells characterized by at least one of elevated expression of CD62L, elevated migration response, elevated homing and in-vivo retention, greater proliferation, increased cytotoxic activity, and a reduced ratio of CD3+ to CD56+/CD3-cells, as compared to a population of NK cells cultured under otherwise identical culturing conditions with less than 0.1 mM of the nicotinamide. In some embodiments, the population of NK cells is characterized by at least any two, at least any three, at least any four, at least any five or all six of elevated expression of CD62L, elevated migration response, elevated homing and in-vivo retention, greater proliferation, increased cytotoxic activity, and a reduced ratio of CD3+ to CD56+/CD3- cells, as compared to a population of NK cells cultured under otherwise identical culturing conditions with less than 0.1 mM of the nicotinamide.

In Example VI the inventors have shown that NK populations prepared according to the methods of the invention have increased *in-vivo* functional potential, as demonstrated by localization and in-vivo retention in the target organs (e.g., spleen, bone marrow and peripheral blood). Thus, in a particular aspect of some embodiments of the present invention there is provided a population of NK cells characterized by enhanced homing, engraftment and in-vivo retention when transplanted, wherein infusion of at least 15X10⁶ cells of the NK cell population into an irradiated host (e.g., a SCID mouse) results in at least 5 %, at least 10 %, at least 15 %, at least 20 %, at least 25 %, at least 30 %, at least 35 %, at least 40 %, at least 45 %, at least 50 %, at least 55 %, at least 60 %, at least 70 %, at least 80 % and at least 90 % or more donor-derived NK cells in the host lymphoid tissue, as detected by immunodetection and flow cytometry, at 4 days post-infusion. In one embodiment, infusion at least 15X10⁶ cells of the NK population results in at least 25 % donor-derived NK cells in the host lymphoid tissue, as detected by immunodetection and flow cytometry, at 4 days post-infusion.

Additional, relevant criteria may be applied for characterizing the NK population. Thus, in yet another embodiment, the NK population of the invention is further characterized by expression of CD62L in at least 30 % of the cell population at the time of infusion into the host, as detected by flow cytometry and immunodetection. In still another embodiment, the NK cell population can be further characterized according to the degree of purity from contamination by CD3+ cells, e.g., an NK cell population having a ratio of CD3+ to CD56+/CD3- cells of equal to or less than 1:100 at the time of infusion.

It will be appreciated, in the context of the present invention, that a therapeutic NK cell population can be provided along with the culture medium containing nicotinamide, isolated from the culture medium, and combined with a pharmaceutically acceptable carrier. Hence, cell populations of the invention can be administered in a pharmaceutically acceptable carrier or diluent, such as sterile saline and aqueous buffer solutions. The use of such carriers and diluents is well known in the art.

In one particular embodiment of this aspect of the present invention, the NK cell population comprises a population of NK cells cultured *ex-vivo* in the presence of an effective amount of nicotinamide; and a pharmaceutically acceptable carrier. In still another embodiment, the *ex-vivo* cultured population comprises NK cells which are activated and have increased cytotoxic capacity to a target cell, when compared to populations of NK cells cultured with growth factors in less than 0.1 mM of the nicotinamide.

The ability of nicotinamide to maintain NK cell proliferation and functionality can be further used in various technical applications:
According to a further aspect of the present invention there is provided a method of preserving NK cells. In one embodiment, the method is effected by handling the NK cells in at least one of the following steps: harvest, isolation and/or storage, in a presence of an effective amount of nicotinamide.

According to still a further aspect of the present invention there is provided a NK cells collection/culturing bag. The cells collection/culturing bag of the present invention is supplemented with an effective amount of nicotinamide.

According to the present invention there is also provided a NK cell separation and/or washing buffer. The separation and/or washing buffer is supplemented with an effective amount of nicotinamide.

As is further detailed below, NK cells may be genetically modified.

In ex-vivo gene therapy cells are removed from a patient, and while being cultured are treated in-vitro. Generally, a functional replacement gene is introduced into the cells via an appropriate gene delivery vehicle/method (transfection, transduction, homologous recombination, etc.) and an expression system as needed and then the modified cells are cultured and returned to the host/patient. These genetically re-implanted cells have been shown to express the transfected genetic material in situ.

Hence, further according to an aspect of the present invention, there is provided a method of transducing ex-vivo cultured NK cells with an exogene. The method, according to this aspect of the present invention, is effected by: (a) ex-vivo culturing a population of NK cells by culturing the population of NK cells according to the methods of NK cell culture of the present invention, and (b) transducing cells of the cultured population of NK cells with the exogene. It will be appreciated that the order of steps (a) and (b) can be reversed. Methods for transduction of cultured NK cells are known in the art, for example, the use of *ex-vivo* modified NK cells has been disclosed by Campana et al. (US20090011498).

Accordingly, the cultured cells of the present invention can be modified to express a gene product. As used herein, the phrase "gene product" refers to proteins, peptides and functional RNA molecules. Generally, the gene product encoded by the nucleic acid molecule is the desired gene product to be supplied to a subject. Examples of such gene products include proteins, peptides, glycoproteins and lipoproteins normally produced by a cell of the recipient subject. Alternatively, the encoded gene product is one, which induces the expression of the desired gene product by the cell (e.g., the introduced genetic material encodes a transcription factor, which induces the transcription of the gene product to be supplied to the subject). For example, the NK cells can be modified to express cell surface molecules, or intracellular gene products which can enhance or modulate NK cell function, such as cytokines, adhesion molecules, activating and/or inhibitory receptors, and the like.

Description of suitable vectors, constructs and protocols for transfection of eukaryotic cells can be found in Current Protocols in Molecular Biology, Ausubel, F.M. et al. (eds.) Greene Publishing Associates (1989), Section 9.2 and in Molecular Cloning: A Laboratory Manual, 2nd Edition, Sambrook et al. Cold Spring Harbor Laboratory Press, (1989), for example, Sections 16.41-16.55, 9 or other standard laboratory manuals.

As is discussed in detail hereinabove, *ex-vivo* propagation of NK cells can be advantageously utilized in NK cells transplantation or implantation. Hence, according to another aspect of the present invention there is provided a method of NK cells transplantation or implantation into a recipient. The method according to this aspect of the present invention is effected by (a) *ex-vivo* culturing a population of NK cells with growth factors and an effective concentration of nicotinamide according to the methods of the invention and administering a therapeutic amount of said cultured NK cells to said subject.

The donor and the recipient can be the same individual or different individuals, for example, allogeneic individuals. Thus, the population of NK cells can be autologous or allogeneic to the subject. When allogeneic transplantation is practiced, regimes for reducing implant rejection and/or graft vs. host disease, as well known in the art, can be undertaken. Such regimes are currently practiced in human therapy. Most advanced regimes are disclosed in publications by Slavin S. et al., e.g., J Clin Immunol (2002) 22: 64, and J Hematother Stem Cell Res (2002) 11: 265), Gur H. et al. (Blood (2002) 99: 4174), and Martelli MF et al., (Semin Hematol (2002) 39: 48)

According to one embodiment, transplantation of the NK cell population is for treatment or prevention of a disease in the subject.

According to yet another aspect of one embodiment of the present invention there is provided a method of inhibiting tumor growth in a subject in need thereof. The method according to this aspect of the present invention is effected by administering a therapeutically effective amount of a population of NK cells of the invention to said subj ect.

"Treating" or "treatment" includes, but is not limited to the administration of an enriched, activated or cultured NK cell composition or population of the present invention to prevent or delay the onset of the symptoms, complications, or biochemical indicia of a disease, alleviating the symptoms or arresting or inhibiting further development of the disease, condition, or disorder (e.g., cancer, metastatic cancer, or metastatic solid tumors). Treatment can be prophylactic, i.e., adjuvant (to prevent or delay the onset of the disease, or to prevent the manifestation of clinical or subclinical symptoms thereof) or therapeutic suppression or alleviation of symptoms after the manifestation of the disease.

In one embodiment, the NK cell population is administered in an amount effective to reduce or eliminate a cancer, such as a solid tumor or a malignancy, or prevent its occurrence or recurrence. "An amount effective to reduce or eliminate the solid tumor or to prevent its occurrence or recurrence" or "an amount effective to reduce or eliminate the hyperproliferative disorder or to prevent its occurrence or recurrence" refers to an amount of a therapeutic composition that improves a patient outcome or survival following treatment for the tumor disease state or hyperproliferative disorder as measured by patient test data, survival data, elevation or suppression of tumor marker levels, reduced susceptibility based upon genetic profile or exposure to environmental factors. "Inhibiting tumor growth" refers to reducing the size or viability or number of cells of a tumor. "Cancer", "malignancy", "solid tumor" or "hyperproliferative disorder" are used as synonymous terms and refer to any of a number of diseases that are characterized by uncontrolled, abnormal proliferation of cells, the ability of affected cells to spread locally or through the bloodstream and lymphatic system to other parts of the body (i.e., metastasize) as well as any of a number of characteristic structural and/or molecular features. A "cancerous" or "malignant cell" or "solid tumor cell" is understood as a cell having specific structural properties, lacking differentiation and being capable of invasion and metastasis. "Cancer" refers to all types of cancer or neoplasm or malignant tumors found in mammals, including carcinomas and sarcomas. Examples are cancers of the breast, lung, non-small cell lung, stomach, brain, head and neck, medulloblastoma, bone, liver, colon, genitourinary, bladder, urinary, kidney, testes, uterus, ovary, cervix, prostate, melanoma, mesothelioma, sarcoma, (see DeVita, et al., (eds.), 2001, Cancer Principles and Practice of Oncology, 6th. Ed., Lippincott Williams & Wilkins, Philadelphia, Pa.).

"Cancer-associated" refers to the relationship of a nucleic acid and its expression, or lack thereof, or a protein and its level or activity, or lack thereof, to the onset of malignancy in a subject cell. For example, cancer can be associated with expression of a particular gene that is not expressed, or is expressed at a lower level, in a normal healthy cell. Conversely, a cancer-associated gene can be one that is not expressed in a malignant cell (or in a cell undergoing transformation), or is expressed at a lower level in the malignant cell than it is expressed in a normal healthy cell.

"Hyperproliferative disease" refers to any disease or disorder in which the cells proliferate more rapidly than normal tissue growth. Thus, a hyperproliferating cell is a cell that is proliferating more rapidly than normal cells.

"Advanced cancer" means cancer that is no longer localized to the primary tumor site, or a cancer that is Stage III or IV according to the American Joint Committee on Cancer (AJCC).

"Well tolerated" refers to the absence of adverse changes in health status that occur as a result of the treatment and would affect treatment decisions.

"Metastatic" refers to tumor cells, e.g., human solid tumor or genitourinary malignancy, that are able to establish secondary tumor lesions in the lungs, liver, bone or brain of immune deficient mice upon injection into the mammary fat pad and/or the circulation of the immune deficient mouse.

A "solid tumor" includes, but is not limited to, sarcoma, melanoma, carcinoma, or other solid tumor cancer. "Sarcoma" refers to a tumor which is made up of a substance like the embryonic connective tissue and is generally composed of closely packed cells embedded in a fibrillar or homogeneous substance. Sarcomas include, but are not limited to, chondrosarcoma, fibrosarcoma, lymphosarcoma, melanosarcoma, myxosarcoma, osteosarcoma, Abemethy's sarcoma, adipose sarcoma, liposarcoma, alveolar soft part sarcoma, ameloblastic sarcoma, botryoid sarcoma, chloroma sarcoma, chorio carcinoma, embryonal sarcoma, Wilms' tumor sarcoma, endometrial sarcoma, stromal sarcoma, Ewing's sarcoma, fascial sarcoma, fibroblastic sarcoma, giant cell sarcoma, granulocytic sarcoma, Hodgkin's sarcoma, idiopathic multiple pigmented hemorrhagic sarcoma, immunoblastic sarcoma of B cells, lymphoma, immunoblastic sarcoma of T-cells, Jensen's sarcoma, Kaposi's sarcoma, Kupffer cell sarcoma, angiosarcoma, leukosarcoma, malignant mesenchymoma sarcoma, parosteal sarcoma, reticulocytic sarcoma, Rous sarcoma, serocystic sarcoma, synovial sarcoma, and telangiectaltic sarcoma.

"Melanoma" refers to a tumor arising from the melanocytic system of the skin and other organs. Melanomas include, for example, acral-lentiginous melanoma, amelanotic melanoma, benign juvenile melanoma, Cloudman's melanoma, S91 melanoma, Harding-Passey melanoma, juvenile melanoma, lentigo maligna melanoma, malignant melanoma, nodular melanoma, subungal melanoma, and superficial spreading melanoma.

"Carcinoma" refers to a malignant new growth made up of epithelial cells tending to infiltrate the surrounding tissues and give rise to metastases. Exemplary carcinomas include, for example, acinar carcinoma, acinous carcinoma, adenocystic carcinoma, adenoid cystic carcinoma, carcinoma adenomatosum, carcinoma of adrenal cortex, alveolar carcinoma, alveolar cell carcinoma, basal cell carcinoma, carcinoma basocellulare, basaloid carcinoma, basosquamous cell carcinoma, bronchioalveolar carcinoma, bronchiolar carcinoma, bronchogenic carcinoma, cerebriform carcinoma, cholangiocellular carcinoma, chorionic carcinoma, colloid carcinoma, comedo carcinoma, corpus carcinoma, cribriform carcinoma, carcinoma en cuirasse, carcinoma cutaneum, cylindrical carcinoma, cylindrical cell carcinoma, duct carcinoma, carcinoma durum, embryonal carcinoma, encephaloid carcinoma, epiermoid carcinoma, carcinoma epitheliale adenoides, exophytic carcinoma, carcinoma ex ulcere, carcinoma fibrosum, gelatiniform carcinoma, gelatinous carcinoma, giant cell carcinoma, carcinoma gigantocellulare, glandular carcinoma, granulosa cell carcinoma, hair-matrix carcinoma, hematoid carcinoma, hepatocellular carcinoma, Hurthle cell carcinoma, hyaline carcinoma, hypemephroid carcinoma, infantile embryonal carcinoma, carcinoma in situ, intraepidermal carcinoma, intraepithelial carcinoma, Krompecher's carcinoma, Kulchitzky-cell carcinoma, large-cell carcinoma, lenticular carcinoma, carcinoma lenticulare, lipomatous carcinoma, lymphoepithelial carcinoma, carcinoma medullare, medullary carcinoma, melanotic carcinoma, carcinoma molle, mucinous carcinoma, carcinoma muciparum, carcinoma mucocellulare, mucoepidernoid carcinoma, carcinoma mucosum, mucous carcinoma, carcinoma myxomatodes, naspharyngeal carcinoma, oat cell carcinoma, carcinoma ossificans, osteoid carcinoma, papillary carcinoma, periportal carcinoma, preinvasive carcinoma, prickle cell carcinoma, pultaceous carcinoma, renal cell carcinoma of kidney, reserve cell carcinoma, carcinoma sarcomatodes, schneiderian carcinoma, scirrhous carcinoma, carcinoma scroti, signet-ring cell carcinoma, carcinoma simplex, small-cell carcinoma, solanoid carcinoma, spheroidal cell carcinoma, spindle cell carcinoma, carcinoma spongiosum, squamous carcinoma, squamous cell carcinoma, string carcinoma, carcinoma telangiectaticum, carcinoma telangiectodes, transitional cell carcinoma, carcinoma tuberosum, tuberous carcinoma, verrucous carcinoma, and carcinoma viflosum.

"Leukemia" refers to progressive, malignant diseases of the blood-forming organs and is generally characterized by a distorted proliferation and development of leukocytes and their precursors in the blood and bone marrow. Leukemia is generally clinically classified on the basis of (1) the duration and character of the disease--acute or chronic; (2) the type of cell involved; myeloid (myelogenous), lymphoid (lymphogenous), or monocytic; and (3) the increase or non-increase in the number of abnormal cells in the blood--leukemic or aleukemic (subleukemic). Leukemia includes, for example, acute nonlymphocytic leukemia, chronic lymphocytic leukemia, acute granulocytic leukemia, chronic granulocytic leukemia, acute promyelocytic leukemia, adult T-cell leukemia, aleukemic leukemia, a leukocythemic leukemia, basophylic leukemia, blast cell leukemia, bovine leukemia, chronic myelocytic leukemia, leukemia cutis, embryonal leukemia, eosinophilic leukemia, Gross' leukemia, hairy-cell leukemia, hemoblastic leukemia, hemocytoblastic leukemia, histiocytic leukemia, stem cell leukemia, acute monocytic leukemia, leukopenic leukemia, lymphatic leukemia, lymphoblastic leukemia, lymphocytic leukemia, lymphogenous leukemia, lymphoid leukemia, lymphosarcoma cell leukemia, mast cell leukemia, megakaryocytic leukemia, micromyeloblastic leukemia, monocytic leukemia, myeloblastic leukemia, myelocytic leukemia, myeloid granulocytic leukemia, myelomonocytic leukemia, Naegeli leukemia, plasma cell leukemia, plasmacytic leukemia, promyelocytic leukemia, Rieder cell leukemia, Schilling's leukemia, stem cell leukemia, subleukemic leukemia, and undifferentiated cell leukemia. Additional cancers include, for example, Hodgkin's Disease, Non-Hodgkin's Lymphoma, multiple myeloma, neuroblastoma, breast cancer, ovarian cancer, lung cancer, rhabdomyosarcoma, primary thrombocytosis, primary macroglobulinemia, small-cell lung tumors, primary brain tumors, stomach cancer, colon cancer, malignant pancreatic insulanoma, malignant carcinoid, urinary bladder cancer, premalignant skin lesions, testicular cancer, lymphomas, thyroid cancer, neuroblastoma, esophageal cancer, genitourinary tract cancer, malignant hypercalcemia, cervical cancer, endometrial cancer, adrenal cortical cancer, and prostate cancer.

In another particular embodiment of this aspect of the present invention the method is affected concomitantly with, following or prior to hematopoietic, hematopoietic progenitor or hematopoietic stem cell transplantation into said subject. In yet further embodiments, the subject is being concomitantly treated with a sensitizing or potentiating agent (e.g., proteasome inhibitor, IL-2, IL-15, etc) further enhancing the *in-vivo* function of the transfused NK cells (for details see, for example, US Patent Application 20090104170 to Childs et al).

Decreased numbers and functionality of NK cells in autoimmune patients has been observed, indicating the possibility of NK cell therapy in a variety of autoimmune diseases and conditions (see Schleinitz, et al., Immunology 2010; 131:451-58, and French and Yokohama, Arthrit Res Ther 2004;6:8-14). Thus, in still another embodiment of the present invention there is provided a method of treating an autoimmune disease or condition in a subject in need thereof. The method according to this aspect of the present invention is effected by administering a therapeutic amount of a population of NK cells of the invention to said subject.

Autoimmune diseases which can be treated by the method of the invention include, but are not limited to cardiovascular diseases, rheumatoid diseases, glandular diseases, gastrointestinal diseases, cutaneous diseases, hepatic diseases, neurological diseases, muscular diseases, nephric diseases, diseases related to reproduction, connective tissue diseases and systemic diseases.

Examples of autoimmune cardiovascular diseases include, but are not limited to atherosclerosis, myocardial infarction, thrombosis, Wegener's granulomatosis, Takayasu's arteritis, Kawasaki syndrome, anti-factor VIII autoimmune disease, necrotizing small vessel vasculitis, microscopic polyangiitis, Churg and Strauss syndrome, pauci-immune focal necrotizing and crescentic glomerulonephritis, antiphospholipid syndrome, antibody-induced heart failure, thrombocytopenic purpura, autoimmune hemolytic anemia, cardiac autoimmunity in Chagas' disease and anti-helper T lymphocyte autoimmunity.

Examples of autoimmune rheumatoid diseases include, but are not limited to rheumatoid arthritis and ankylosing spondylitis.

Examples of autoimmune glandular diseases include, but are not limited to, pancreatic disease, Type I diabetes, thyroid disease, Graves' disease, thyroiditis, spontaneous autoimmune thyroiditis, Hashimoto's thyroiditis, idiopathic myxedema, ovarian autoimmunity, autoimmune anti-sperm infertility, autoimmune prostatitis and Type I autoimmune polyglandular syndrome. diseases include, but are not limited to autoimmune diseases of the pancreas, Type 1 diabetes, autoimmune thyroid diseases, Graves' disease, spontaneous autoimmune thyroiditis, Hashimoto's thyroiditis, idiopathic myxedema, ovarian autoimmunity, autoimmune anti-sperm infertility, autoimmune prostatitis and Type I autoimmune polyglandular syndrome.

Examples of autoimmune gastrointestinal diseases include, but are not limited to, chronic inflammatory intestinal diseases, celiac disease, colitis, ileitis and Crohn's disease.

Examples of autoimmune cutaneous diseases include, but are not limited to, autoimmune bullous skin diseases, such as, but are not limited to, pemphigus vulgaris, bullous pemphigoid and pemphigus foliaceus.

Examples of autoimmune hepatic diseases include, but are not limited to, hepatitis, autoimmune chronic active hepatitis, primary biliary cirrhosis and autoimmune hepatitis.

Examples of autoimmune neurological diseases include, but are not limited to, multiple sclerosis, Alzheimer's disease, myasthenia gravis, neuropathies, motor neuropathies; Guillain-Barre syndrome and autoimmune neuropathies, myasthenia, Lambert-Eaton myasthenic syndrome; paraneoplastic neurological diseases, cerebellar atrophy, paraneoplastic cerebellar atrophy and stiff-man syndrome; non-paraneoplastic stiff man syndrome, progressive cerebellar atrophies, encephalitis, Rasmussen's encephalitis, amyotrophic lateral sclerosis, Sydeham chorea, Gilles de la Tourette syndrome and autoimmune polyendocrinopathies; dysimmune neuropathies; acquired neuromyotonia, arthrogryposis multiplex congenita, neuritis, optic neuritis and neurodegenerative diseases.

Examples of autoimmune muscular diseases include, but are not limited to, myositis, autoimmune myositis and primary Sjogren's syndrome and smooth muscle autoimmune disease.

Examples of autoimmune nephric diseases include, but are not limited to, nephritis and autoimmune interstitial nephritis.

Examples of autoimmune diseases related to reproduction include, but are not limited to, repeated fetal loss.

Examples of autoimmune connective tissue diseases include, but are not limited to, ear diseases, autoimmune ear diseases and autoimmune diseases of the inner ear.

Examples of autoimmune systemic diseases include, but are not limited to, systemic lupus erythematosus and systemic sclerosis.

In yet another embodiment of the present invention there is provided a method of inhibiting a viral infection in a subject in need thereof. The method according to this aspect of the present invention is effected by (a) *ex-vivo* culturing a population of NK cells with NK cell growth factors and an effective concentration of nicotinamide, wherein said effective concentration of nicotinamide enhances proliferation of said NK cells, as compared to said population of cells cultured with growth factors without said concentration of nicotinamide; and (b) administering a therapeutic amount of said cultured NK cells to said subject. Viral infections suitable for treatment with NK cells or NK cell compositions of the invention include, but are not limited to HIV, lymphatic choriomenengitis virus (LCMV), cytomegalovirus (CMV), vaccinia virus, influenza and para-influenza virus, hepatitis (including hepatitis A, hepatitis B, hepatitis C, non-Anon-B, etc), herpes simplex virus, herpes zoster virus, Theiler's virus and HSV-1. Other infectious diseases suitable for treatment with NK cells or NK cell preparations of the present invention include, but are not limited to parasitic infections such as Plasmodium, Leishmania and Toxiplasma infections, and bacterial infections such as mycobacteria and Listeria (for a review of NK cells in treatment of viral, bacterial and protozoan diseases see Zucchini et al., Exp Rev Anti-Infect Ther 2008;6:867-85).

Transplantation of hematopoietic cells has become the treatment of choice for a variety of inherited or malignant diseases. However, hematopoietic cell compositions are often rich in T lymphocytes, which contribute to graft-versus-host disease. Since patients suffering from hematological malignancies are often deficient in NK cell numbers and function, exogenous administration NK cells along with hematopoietic cell transplantation is currently being investigated for enhanced long term engraftment and prevention of graft versus host disease. Thus, in yet another embodiment of the present invention there is provided a method of treating or preventing graft versus host disease in a subject in need thereof. Thus, in still another embodiment of the present invention there is provided a method of treating an autoimmune disease or condition in a subject in need thereof. The method according to this aspect of the present invention is effected by administering a therapeutic amount of a population of NK cells of the invention to said subject.

Clinical protocols for treatment with NK cells, and combinations treatments with NK and HSC cells populations are well known in the art. For example, recent reports have established that NK cells infusions are safe, and do not cause GVHD in the recipient. One such protocol involves myeloablation, infusion of IL-2 activated, NK enriched (non-NK depleted) HLA-mismatched cord blood, followed by a double cord blood infusion for HSC repopulation [see Miller et al., Blood 2006; 108:3111 (Abstract)]. The authors reported that transplantation of NK cells, along with cord blood HSC, resulted in improved long-term engraftment of the HSC.

### Treatment Regimes

According to some aspects of some embodiments of the present invention, there are provided pharmaceutical compositions comprising an NK cell population for the treatment of disease, e.g., metastic cancer, solid tumors, autoimmune disease, hyperproliferative disorder or a viral infection, formulated together with a pharmaceutically acceptable carrier. Some compositions include a combination of multiple (e.g., two or more) NK cell populations of the invention.

In prophylactic applications, pharmaceutical compositions or medicaments are administered to a patient susceptible to, or otherwise at risk of a disease or condition (i.e., a hyperproliferative disease or solid tumor) in an amount sufficient to eliminate or reduce the risk of recurrence of the hyperproliferative disease or solid tumor, lessen the severity, or delay the outset of the disease, including biochemical, histologic and/or behavioral symptoms of the disease, its complications and intermediate pathological phenotypes presenting during development of the disease. In therapeutic applications, compositions or medicants are administered to a patient suspected of, or already suffering from such a disease in an amount sufficient to cure, or at least partially arrest, the symptoms of the disease (biochemical, histologic and/or behavioral), including its complications and intermediate pathological phenotypes in development of the disease. An amount adequate to accomplish therapeutic or prophylactic treatment is defined as a therapeutically- or prophylactically-effective dose. In both prophylactic and therapeutic regimes, agents are usually administered in several dosages until a sufficient anti-proliferative response has been achieved. Typically, the anti-proliferative response is monitored and repeated dosages are given if the anti-proliferative response starts to wane.

### Effective Dosages

Effective doses of a composition of an NK cell population for the treatment of disease, e.g., metastic cancer, solid tumors, or a hyperproliferative disorder, described herein vary depending upon many different factors, including means of administration, target site, physiological state of the patient, whether the patient is human or an animal, other medications administered, and whether treatment is prophylactic or therapeutic. Usually, the patient is a human but nonhuman mammals including transgenic mammals can also be treated. Treatment dosages need to be titrated to optimize safety and efficacy.

For administration with a therapeutic NK cell population, the dosage ranges from about 1X10⁶ to about 1X10⁹ NK cells per patient. For administration with an NK cell population, the dosage ranges from about 1X10⁵ to about 1X10⁹ NK cells per kilogram recipient weight, or the dosage ranges from about 5X10⁵ to about 1X10⁸ NK cells per kilogram recipient weight. An exemplary treatment regime entails administration once per every two weeks or once a month or once every 3 to 6 months. In some methods, two or more NK cell populations are administered simultaneously, in which case the dosage of each NK cell populations administered falls within the ranges indicated. Multiple administrations of NK cell populations can occur. Intervals between single dosages can be weekly, monthly or yearly. Intervals can also be irregular as indicated by measuring blood levels of the NK cell population in the patient. Alternatively, the NK cell populations can be administered as a sustained release formulation, in which case less frequent administration is required. Dosage and frequency vary depending on the half-life of the NK cell populations in the patient. The dosage and frequency of administration can vary depending on whether the treatment is prophylactic or therapeutic. In prophylactic applications, a relatively low dosage is administered at relatively infrequent intervals over a long period of time. Some patients continue to receive treatment for the rest of their lives. In therapeutic applications, a relatively high dosage at relatively short intervals is sometimes required until progression of the disease is reduced or terminated, and preferably until the patient shows partial or complete amelioration of symptoms of disease. Thereafter, the patent can be administered a prophylactic regime.

### Routes of Administration

Compositions of a therapeutic NK cell population for the treatment of disease, e.g., metastic cancer, solid tumors, or a hyperproliferative disorder, can be administered by intravenous, intravesicular, intrathecal, parenteral, topical, subcutaneous, oral, intranasal, intraarterial, intracranial, intraperitoneal, or intramuscular means. As a prophylactic/adjuvant or for treatment of disease, therapeutic NK cell populations target a hyperproliferative disorder or solid tumor, e.g., a genitourinary malignancy, and/or therapeutic treatment. The most typical route of administration of an immunogenic agent is subcutaneous although other routes can be equally effective. The next most common route is intramuscular injection. This type of injection is most typically performed in the arm or leg muscles. In some methods, agents are injected directly into a particular tissue where deposits have accumulated, for example intracranial injection. Intramuscular injection on intravenous infusion are preferred for administration of an NK cell population. In some methods, a particular therapeutic NK cell population is injected directly into the bladder.

### Formulation

Compositions of an NK cell population for the treatment of disease, e.g., metastic cancer, solid tumors, viral infection, or a hyperproliferative disorder.

Compositions of a therapeutic NK cell population for the treatment of disease, e.g., metastic cancer, solid tumors, or a hyperproliferative disorder, are often administered as pharmaceutical compositions comprising an active therapeutic agent, i.e., and a variety of other pharmaceutically acceptable components. See, e.g., Alfonso R Gennaro (ed), Remington: The Science and Practice of Pharmacy, (Formerly Remington's Pharmaceutical Sciences) 20th ed., Lippincott, Williams & Wilkins, 2003. The preferred form depends on the intended mode of administration and therapeutic application. The compositions can also include, depending on the formulation desired, pharmaceutically-acceptable, non-toxic carriers or diluents, which are defined as vehicles commonly used to formulate pharmaceutical compositions for animal or human administration. The diluent is selected so as not to affect the biological activity of the combination. An example of such diluent is X-vivo 20 media (Cambrex Bio Science, Walkersville, Md.) containing 10 % heat inactivated human AB serum or 10 % autologous serum. Further examples of such diluents are distilled water, physiological phosphate-buffered saline, Ringer's solutions, dextrose solution, and Hank's solution. In addition, the pharmaceutical composition or formulation can also include other carriers, adjuvants, or nontoxic, nontherapeutic, nonimmunogenic stabilizers and the like.

Pharmaceutical compositions can also include large, slowly metabolized macromolecules such as proteins, polysaccharides such as chitosan, polylactic acids, polyglycolic acids and copolymers (such as latex functionalized Sepharose.™., agarose, cellulose, and the like), polymeric amino acids, amino acid copolymers, and lipid aggregates (such as oil droplets or liposomes). Additionally, these carriers can function as immunostimulating agents (i.e., adjuvants).

For parenteral administration, compositions of the invention can be administered as injectable dosages of a solution or suspension of the substance in a physiologically acceptable diluent with a pharmaceutical carrier that can be a sterile liquid such as water oils, saline, glycerol, or ethanol. Additionally, auxiliary substances, such as wetting or emulsifying agents, surfactants, pH buffering substances and the like can be present in compositions. Other components of pharmaceutical compositions are those of petroleum, animal, vegetable, or synthetic origin, for example, peanut oil, soybean oil, and mineral oil. In general, glycols such as propylene glycol or polyethylene glycol are preferred liquid carriers, particularly for injectable solutions. Therapeutic NK cell populations can be administered in the form of a depot injection or implant preparation which can be formulated in such a manner as to permit a sustained release of the active ingredient. An exemplary composition comprises a therapeutic NK cell population at 5 mg/mL, formulated in aqueous buffer consisting of 50 mM L-histidine, 150 mM NaCl, adjusted to pH 6.0 with HCl.

Typically, compositions are prepared as injectables, either as liquid solutions or suspensions; solid forms suitable for solution in, or suspension in, liquid vehicles prior to injection can also be prepared. The preparation also can be emulsified or encapsulated in liposomes or micro particles such as polylactide, polyglycolide, or copolymer for enhanced adjuvant effect, as discussed above. Langer, Science, 249: 1527, 1990; Hanes, Advanced Drug Delivery Reviews, 28: 97-119, 1997. The agents of this invention can be administered in the form of a depot injection or implant preparation which can be formulated in such a manner as to permit a sustained or pulsatile release of the active ingredient. Additional formulations suitable for other modes of administration include oral, intranasal, and pulmonary formulations, suppositories, and transdermal applications.

For suppositories, binders and carriers include, for example, polyalkylene glycols or triglycerides; such suppositories can be formed from mixtures containing the active ingredient in the range of 0.5 % to 10 %, preferably 1 %-2 %. Oral formulations include excipients, such as pharmaceutical grades of mannitol, lactose, starch, magnesium stearate, sodium saccharine, cellulose, and magnesium carbonate. These compositions take the form of solutions, suspensions, tablets, pills, capsules, sustained release formulations or powders and contain 10 %-95 % of active ingredient, preferably 25 %-70 %.

The pharmaceutical compositions generally comprise a composition of the therapeutic NK cell population in a form suitable for administration to a patient. The pharmaceutical compositions are generally formulated as sterile, substantially isotonic and in fall compliance with all Good Manufacturing Practice (GMP) regulations of the U.S. Food and Drug Administration.

### Toxicity

Preferably, a therapeutically effective dose of a composition of the NK cell population described herein will provide therapeutic benefit without causing substantial toxicity.

Toxicity of the therapeutic NK cell population described herein can be determined by standard pharmaceutical procedures in cell cultures or experimental animals, e.g., by determining the LD.sub.50 (the dose lethal to 50 % of the population) or the LD.sub. 100 (the dose lethal to 100 % of the population). The dose ratio between toxic and therapeutic effect is the therapeutic index. The data obtained from these cell culture assays and animal studies can be used in formulating a dosage range that is not toxic for use in human. The dosage of the theraeputic NK cell population described herein lies preferably within a range of circulating concentrations that include the effective dose with little or no toxicity. The dosage can vary within this range depending upon the dosage form employed and the route of administration utilized. The exact formulation, route of administration and dosage can be chosen by the individual physician in view of the patient's condition. (See, e.g., Fingl, et al., The Pharmacological Basis Of Therapeutics, Ch. 1, 1975).

### Kits

Also within the scope of the invention are kits comprising the compositions (e.g., a therapeutic NK cell population) of the invention and instructions for use. The kit can further contain a least one additional reagent, or one or more additional human antibodies of the invention (e.g., a human antibody having a complementary activity which binds to an epitope in the antigen distinct from the first human antibody). Kits typically include a label indicating the intended use of the contents of the kit. The term label includes any writing, or recorded material supplied on or with the kit, or which otherwise accompanies the kit.

As used herein the term "about" refers to ± 10 %.

The terms "comprises", "comprising", "includes", "including", "having" and their conjugates mean "including but not limited to". This term encompasses the terms "consisting of" and "consisting essentially of".

The phrase "consisting essentially of" means that the composition or method may include additional ingredients and/or steps, but only if the additional ingredients and/or steps do not materially alter the basic and novel characteristics of the claimed composition or method.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples, which together with the above descriptions, illustrate some embodiments of the invention in a non-limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1, 2, 317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996). Other general references are provided throughout this document. The procedures therein are believed to be well known in the art and are provided for the convenience of the reader.

### Materials and Experimental procedures

### Cord blood samples

Cells were obtained from umbilical cord blood after normal full-term delivery (informed consent was given). Samples were collected and frozen within 24 hours postpartum. Briefly, cord blood was collected by gravity from delivered placentas, the leukocyte-rich fraction was separated by density gradient centrifugation, cells mixed with DMSO (10 %) and then frozen at -80 °C. Prior to use, the cells were thawed in Dextran buffer (Sigma, St. Louis, MO, USA) containing 2.5 % human serum albumin (HSA)(Bayer Corp. Elkhart, IN, USA), and the cryoprotectant removed.

### BM and peripheral blood samples

Bone marrow (BM) and peripheral blood (PB) cells were layered on a Ficoll-Hypaque gradient (1.077 g/mL; Sigma), and centrifuged at 800xg for 30 min. The mononuclear cells in the interface layer were collected and washed three times in phosphate-buffered saline (PBS) (Biological Industries, Israel) containing 0.5 % HSA.

### Enrichment of CD56+ cells

Cord blood (CB), bone marrow (BM) or peripheral blood (PB) cells were layered on a Ficoll-Hypaque gradient (1.077 g/mL; Sigma), and centrifuged at 800xg for 30 minutes for separation of the mononuclear cells. The cells in the interface layer were collected and washed three times in phosphate-buffered saline (PBS) (Biological Industries, Israel) containing 0.5 % HSA. To purify the CD56+ cells, the mononuclear cell fraction was subjected to two cycles of immunomagnetic bead separation, using a "MiniMACS or CliniMACS CD56 progenitor cell isolation kit" (Miltenyi Biotec Bergisch, Gladbach, Germany), according to the manufacturer's recommendations. Briefly, CD56+ cells are reacted with CD56+ specific magnetic immunobeads, separated and with a magnetic separator, and purified from unbound cells by washing. The purity of the CD56+ population thus obtained is approximately 92 %, as evaluated by flow cytometry.

Optionally, cells are not separated on the Ficoll-Hypaque gradient but washed three times in phosphate-buffered saline (PBS) (Biological Industries) containing 0.5 % HSA ("total mononuclear fraction"). In the last wash cells were incubated with 50µg/ml rHu-DNAse for 10 minutes. To purify the CD56+ cells, the cells are subjected to two cycles of immunomagnetic bead separation, using a "MidiMACS CD56 progenitor cell isolation kit" (Miltenyi Biotec Bergisch, Gladbach, Germany), according to the manufacturer's recommendations. The purity of the CD56+ population thus obtained is approximately 92 %, as evaluated by flow cytometry.

Optionally, bone marrow cells are depleted of CD133+ or CD34+ cells by immunomagnetic bead separation, using a "MidiMACS or CliniMACS CD133 cell isolation kit" (Miltenyi Biotec Bergisch, Gladbach, Germany), and then the CD133-or CD34 negative fraction is further enriched for NK cells by subjecting the cells to two cycles of immunomagnetic bead separation, using a "MidiMACS or CliniMACS CD56 progenitor cell isolation kit" (Miltenyi Biotec Bergisch, Gladbach, Germany), according to the manufacturer's recommendations.

### Enrichment of CD56+CD3- cells

Cord blood (CB), bone marrow (BM) or peripheral blood (PB) cells were layered on a Ficoll-Hypaque gradient (1.077 g/mL; Sigma), and centrifuged at 800xg for 30 minutes for separation of the mononuclear cells. The cells in the interface layer were collected and washed three times in phosphate-buffered saline (PBS) (Biological Industries, Israel) containing 0.5 % HSA. To purify the CD56+CD3- cells, the mononuclear cell fraction was subjected to immunomagnetic bead separation, using a "MiniMACS or CliniMACS CD56 progenitor cell isolation kit" (Miltenyi Biotec Bergisch, Gladbach, Germany), according to the manufacturer's recommendations. Briefly, CD56+ cells are reacted with CD56+ specific magnetic immunobeads, separated and with a magnetic separator, and purified from unbound cells by washing. The purified CD56+ cell fraction was subjected to an additional immunomagnetic bead separation, using a "MiniMACS or CliniMACS CD3 progenitor cell isolation kit" (Miltenyi Biotec Bergisch, Gladbach, Germany), according to the manufacturer's recommendations. Briefly, CD56+ cells are reacted with CD3+ specific magnetic immunobeads, separated and with a magnetic separator, and CD56+CD3- cells recovered in the unbound cells fraction. The purity of the CD56+CD3- population thus obtained is approximately 85-97 %, as evaluated by flow cytometry.

Optionally, cells are not separated on the Ficoll-Hypaque gradient but washed three times in phosphate-buffered saline (PBS) (Biological Industries) containing 0.5 % HSA ("total mononuclear fraction"). In the last wash cells were incubated with 50µg/ml rHu-DNAse for 10 minutes. To purify the CD56+ cells, the cells are subjected to two cycles of immunomagnetic bead separation, using a "MidiMACS CD56 progenitor cell isolation kit" (Miltenyi Biotec Bergisch, Gladbach, Germany), according to the manufacturer's recommendations. The purity of the CD56+ population thus obtained is approximately 92 %, as evaluated by flow cytometry. To purify the CD56+CD3- cells, the cells are subjected to immunomagnetic bead separation, using a "MiniMACS or CliniMACS CD56 progenitor cell isolation kit" (Miltenyi Biotec Bergisch, Gladbach, Germany), according to the manufacturer's recommendations. Briefly, CD56+ cells are reacted with CD56+ specific magnetic immunobeads, separated and with a magnetic separator, and purified from unbound cells by washing. The purified CD56+ cell fraction was subjected to an additional immunomagnetic bead separation, using a "MiniMACS or CliniMACS CD3 progenitor cell isolation kit" (Miltenyi Biotec Bergisch, Gladbach, Germany), according to the manufacturer's recommendations. Briefly, CD56+ cells are reacted with CD3+ specific magnetic immunobeads, separated and with a magnetic separator, and CD56+CD3- cells recovered in the unbound cells fraction.The purity of the CD56+CD3- population thus obtained is approximately 85-97 %, as evaluated by flow cytometry.

Optionally, bone marrow cells are depleted of CD133+ or CD34+ cells by immunomagnetic bead separation, using a "MidiMACS or CliniMACS CD133 cell isolation kit" (Miltenyi Biotec Bergisch, Gladbach, Germany), and then the CD133-or CD34 negative fraction is further enriched for NK cells by subjecting the cells to two cycles of immunomagnetic bead separation, using a "MidiMACS or CliniMACS CD56 progenitor cell isolation kit" (Miltenyi Biotec Bergisch, Gladbach, Germany), according to the manufacturer's recommendations. To purify the CD56+CD3- cells, CD133-or CD34 negative fraction was subjected to immunomagnetic bead separation, using a "MiniMACS or CliniMACS CD56 progenitor cell isolation kit" (Miltenyi Biotec Bergisch, Gladbach, Germany), according to the manufacturer's recommendations. Briefly, CD56+ cells are reacted with CD56+ specific magnetic immunobeads, separated and with a magnetic separator, and purified from unbound cells by washing. The purified CD56+ cell fraction was subjected to an additional immunomagnetic bead separation, using a "MiniMACS or CliniMACS CD3 progenitor cell isolation kit" (Miltenyi Biotec Bergisch, Gladbach, Germany), according to the manufacturer's recommendations. Briefly, CD56+ cells are reacted with CD3+ specific magnetic immunobeads, separated and with a magnetic separator, and CD56+CD3- cells recovered in the unbound cells fraction.The purity of the CD56+CD3- population thus obtained is approximately 85-97 %, as evaluated by flow cytometry.

### Depletion of CD3+ or CD3+ CD19+ cells before culture

For depletion procedure, total nuclear cells from umbilical cord blood (CB), bone marrow (BM) or peripheral blood (PB) cells were layered on a Ficoll-Hypaque gradient (1.077 g/mL; Sigma), and centrifuged at 800xg for 30 minutes for separation of the mononuclear cells. The cells in the interface layer were collected and washed three times in phosphate-buffered saline (PBS) (Biological Industries, Israel) containing 0.5 % HSA. Optionally, cells are not separated on the Ficoll-Hypaque gradient but washed three times in phosphate-buffered saline (PBS) (Biological Industries) containing 0.5 % HSA ("total mononuclear fraction"). CD3 cells were depleted using the CD3 cell isolation kit (Miltenyi Biotec Bergisch, Gladbach, Germany) and the entire CD3 negative cell fraction was cultured. Optionally, CD19 cells were also depleted using the CD19 cell isolation kit (Miltenyi Biotec Bergisch, Gladbach, Germany) and the CD3/CD19 negative (CD3/CD19 depleted) cell fraction was cultured. Optionally, RosetteSep Human CD3+ Cell Depletion Cocktail (Stem Cell Technologies, RosestteSep, Cat. No. 15661)] was used for CD3 depletion and the entire CD3 negative cell fraction was cultured. After negative depletion the cells were counted and characterized by FACS analysis.

### Ex vivo cultures:

1. Total mononuclear cell fraction was cultured in culture bags (American Fluoroseal Co. Gaithersburg, MD, USA), T-Flasks or 24 well plates at a concentration of 0.5-2x10⁶ cells/ml in MEMα comprising 10 % FCS or CellGro SCGM (Cell Genix) comprising 5-10 % Human Serum/LiforCell® FBS Replacement (Lifeblood Products) containing the following human recombinant cytokines:, interleukin-2 (IL-2) (5-50ng/ml), interleukin-15 (IL-15), interleukin7 (IL7), interleukin 21 (IL21), FLT-3 or SCF or FLT3 and SCF (Perpo Tech, Inc., Rocky Hill, NJ, USA), with or without OKT-3 (10-50ng/ml), with or without nicotinamide (0.5-10 mM), and incubated at 37°C in a humidified atmosphere of 5 % CO₂ in air. When OKT-3 was used in the culture the cultures were centrifuged after 5-7 days and the cells were resuspended with the same medium excluding the OKT-3. All cultures are topped weekly or twice a week with the same volume of fresh medium containing the growth factors with or without nicotinamide. For counting, the cells were stained with trypan blue. At various time points, samples were taken to assay the relative fractions of NK cells, CD56+CD3-, CD56+CD3+, CD34+CD56+, CD56+CD16+ and/or CD56+NKG2A cells. Cell morphology was determined on cytospin (Shandon, Pittsburgh, PA, USA) prepared smears stained with May-Grunwald/Giemsa solutions.
2. Purified CD56+ or CD56+CD3- cells from total nuclear or mononuclear cells or from the fraction depleted from CD34+ or CD133+ cells were cultured in culture bags, T-Flasks or 24 well plates at a concentration of 1- 100 x10⁴ cells/ml in MEMα/10 % FCS or CellGro SCGM (Cell Genix)/5 % Human Serum/LiforCell® FBS Replacement (Lifeblood Products) containing the following human recombinant cytokines:, interleukin-2 (IL-2) (5-50ng/ml), interleukin-15 (IL-15), FLT-3 or SCF or FLT3 and SCF or IL-2 and IL-15 or IL-2 only (Perpo Tech, Inc., Rocky Hill, NJ, USA), with or without nicotinamide, and incubated at 37°C in a humidified atmosphere of 5 % CO₂ in air. The cultures were topped up once or twice a week with the same volume of fresh medium containing growth factors with or without nicotinamide. Eventually cultures can be supplemented once or several times a week with IL-2 and or IL-2 and IL-15. To estimate the number of cells in culture, cell samples were stained with trypan blue for counting. At various time points, samples were taken for FACS analysis to assay the relative fractions of NK cells, CD56+CD3-, CD56+CD3+, CD34+CD56+, CD56+CD16+ and/or CD56+NKG2A cells.
3. CD3+ depleted, or CD3+/CD19+ depleted mononuclear cell fraction from PB, BM and CB were cultured in culture bags, T-Flasks or 24 well plates at a concentration of 1-1000x10⁴ cells/ml in MEMα/10 % FCS or CellGro SCGM (Cell Genix)/5 % Human Serum/LiforCell® FBS Replacement (Lifeblood Products) containing the following human recombinant cytokines: interleukin-2 (IL-2) or and interleukin-15 (IL-15), with or without FLT-3 or SCF or FLT3 and SCF (5-50ng/ml) (Perpo Tech, Inc., Rocky Hill, NJ, USA), all combinations, with or without nicotinamide and incubated at 37°C in a humidified atmosphere of 5 % CO₂ in air. The cultures were topped weekly or twice a week with the same volume of fresh medium containing growth factors with or without nicotinamide. Cultures were later supplemented once or several times a week with IL-2 and or IL-2 and IL-15. To estimate number of cells in culture, cell samples were counted following staining with trypan blue. At various time points, samples are taken to FACS analysis to assay the relative fractions of NK cells, CD56+CD3-, CD56+CD3+, CD34+CD56+, CD56+CD16+ and/or CD56+NKG2A cells.
4. Total mononuclear cell fraction, purified CD56+ or CD56+CD3- cells from total nuclear or mononuclear cells or from the fraction depleted from CD34+ or CD133+, and CD3+ depleted, or CD3+/CD19+ depleted mononuclear cell fraction from PB, BM and CB cells can be also cultured in a bioreactor such as GE Wave Bioreactor bag or Gas Permeable Cultureware flasks (Wilson Wolf) at 1-10000x10⁴ cells/ml) Culture medium contained MEMα, Human Serum (10 %v/v), 20ng/ml IL-2, and optionally 50ng/ml IL-2 and optionally IL-15, with nicotinamide. The cultures were topped up weekly or twice a week with the same volume of fresh medium containing growth factors with or without nicotinamide. Cultures were later supplemented once or several times a week with IL-2 and or IL-2 and IL-15.and the cells were counted and stained for FACS analysis after 1, 2 and 3 weeks.

### NK cell culture with irradiated stroma

NK cell cells from total mononuclear cell fraction, purified CD56+ or CD56+CD3- cells from total nuclear or mononuclear cells or from the fraction depleted from CD34+ or CD133+ cells, or CD3+ depleted, or CD3+/CD19+ depleted mononuclear cell fraction from PB, BM and CB can be cultured with irradiated stroma. For the preparation of irradiated stroma (feeder cells), mononuclear cells were irradiated with 3000 rad. After CD56 or CD56+CD3- selection or CD3 depletion the cells were counted and characterized by FACS analysis as described hereinabove. Cells were cultured as described hereinabove, with and without nicotinamide, with and without irradiated stroma at a concentration of irradiated cells of 20x10⁵ cells/ml.

### FACS analysis

For FACS analysis cells were stained with the following fluorescent antibodies: CD15 FITC, Cat. No. 332778, CD14 FITC, Cat. No. 345784, CD3 APC, Cat. No. 345767, all from Becton Dickinson (San Jose, CA, USA), CD62L PE, Cat. No. 304806 from BioLegend (San Diego, CA, USA), CD56 FITC, Cat. No.11-0569-42 from eBioscience (San Diego, CA, USA) and CD45 PE, Cat. No. R7807 from Dako (Glostrup, Denmark)

NK cells were also characterized by CXCR4, KIR3, DL1/2, DL2/2, DL1, NKG2A, NKG2C, NKG2D, TRAIL, Fc-gamma receptor IIIb, NKp44, NKp30, NKp46, Fas-L, L-Selectin, phycoerythrin, IL-2 receptor γ chain (CD16), VLA-5α chain and CD8.

### Calculation of number of NK cells seeded at day 0

In order to calculate the number of NK cells on day 0, the total number of cells seeded on day 0 was multiplied by the percent of CD56+/CD3- cells measured by the FACS on day 0.

### Calculation of NK cell number in culture and fold increase 7, 14 and 21 days post seeding

To determine total number of cells on day 7, 14 and 21, the cell count/ml was multiplied by the volume of the culture medium. In order to determine the number of NK cells in culture, the total number of cells in culture was multiplied by the percent of CD56+/CD3- cells measured with the FACS on days 7, 14 or 21. To measure fold expansion, total number of NK cells on days 7, 14 and 21 was divided by total number of NK cells seeded in culture on day-0.

### Surface antigen analysis

The cells were washed with a PBS solution containing 1 % BSA, and stained (at 4°C for 30 min) with fluorescein isothiocyanate (FITC)- or phycoerythrin (PE)-conjugated antibodies or allophycocyanin (APC) . The cells were then washed in the above buffer and analyzed using a FACScalibur® flow cytometer (Becton Dickinson, San Jose, CA, USA). The cells were passed at a rate of up to 1000 cells/second, using a 488 nm or 661nm argon laser beam as the light source for excitation. Emission of 10⁴ cells was measured using logarithmic amplification, and analyzed using the CellQuest software (Becton Dickinson). Cells stained with FITC, PE and APC-conjugated isotype control antibodies were used to determine background fluorescence.

### Determination of the functionality of NK cells

***"Killing" assay:*** NK cells (effectors cells = E) are combined with K562 or BL2 (target cells = T), or bi-phenotypic leukemia cells at different E to T ratio (E:T). BL2 or K562, or bi-phenotypic leukemia target cells in PBS were labeled with 1 ng/ml CFSE (Invitrogen) for 15 min at 37°C. Calf Serum was added to the cells for 15 minutes, the cells were then washed and resuspended in RPMI medium. 100µL of bi-phenotypic leukemia, BL2 or K562 cells were placed in a 96 round bottom plate at a concentration of 5X10³ cells per well. 100µL of non-stained NK cells were added to the bi-phenotypic leukemia, BL2 or K562 cells at a E:T ratio of 1:1, 2.5:1, 5:1, 10:1 or 20:1 (5X10³, 1.25X10⁴, 2.5X10⁴, 5X10⁴ and 1X10⁵ cells/well, respectively, as indicated). Between 2-48 hours later. Killing of target cells in cell lines such as K562 and BL-2 was determined by FACS as a percentage propidium-iodine (PI)-positive (dead) CFSE-labeled cells. Killing of the primary leukemia cells was determined by counting with the FACS the number of CFSE stained cells that remained in the culture after their culture with the NK cells. A lower number of CFSE+ cells is indicative of higher level of killing.

***Chemotaxis ("migration") assay:*** Migration response (chemotaxis) of human NK cells was assayed by Transwell migration assay (Costar, Cambridge, MA; 6.5-mm diameter, 5-µm pore size). Briefly, 100 µL chemotaxis buffer (RPMI 1640, 1 % FCS) containing 2 x10⁵ NK cells was added to the upper chamber, and 0.5 mL chemotaxis buffer with or without 250ng/ml stromal-derived factor CXCL12 ("SDF-1") (R&D Systems) was added to the bottom chamber. Cells migrating within 4 hours to the bottom chamber of the "transwell" were counted for 60 seconds using FACScalibur (Becton Dickinson Immunocytometry Systems).

### "In-vivo" homing and engraftment:

NK cells were expanded with or without 2.5 mM or 5.0 mM nicotinamide, as described above. After 2-3 weeks in culture, similar numbers (15x10⁶) of cells were infused into irradiated (350 Rad) NOD/SCID mice. Mice were sacrificed 4-days post infusion. Spleen, bone marrow and peripheral blood were analyzed for the homing and engraftment of human NK (CD45+CD56+) cells using immunomagnetic beads, in order to distinguish them from endogenous cells. Engraftment is expressed as the % of NK cells having the transplanted lineage (CD45+CD56+) using anti-human specific antibodies from the total number of mouse endogenous cells identified in the tissue.

### Assay of CD62L expression on NK cell surface

Cultures were initiated with peripheral-blood-derived CD56+ cells purified with immunomagnetic beads and activated with cytokines (including IL-2 and IL-15) with or without nicotinamide (2.5, 5 and 7.5 mM). NK cells were stained with specific antibodies for the specified surface markers (e.g., CD62L) before activation in culture and after 3 weeks incubation with nicotinamide, and then monitored by FACS.

### Results

### EXAMPLE I: Nicotinamide enhances ex-vivo propagation of NK cells

In order to evaluate the effect of added nicotinamide on *ex-vivo* growth of NK cells, cord blood or bone marrow cells were incubated with growth factors (cytokines) and increasing concentrations of nicotinamide, without feeder cells or feeder layer, and NK and non-NK (e.g., CD3+) cell fractions measured at different time points.

CD56+ cells derived from cord blood were found to be rich in the CD56+CD3-NK cell population, and contains relatively few CD56+CD3+ NKT cells. When purified cord blood NK cells (CD56+) were incubated with nicotinamide, in the presence of IL-2 and IL-15, significantly enhanced proliferation of NK cells was evident as early as 14 days of culture, and at all concentrations tested. FIG. 1A shows that the proliferation of NK cells with 2.5 mM nicotinamide at 14 days was greater than 4 times that of cells incubated with growth factors ("cytokines") (including IL-2 and IL-15) alone, and even greater with 5 mM nicotinamide. When a broader range of nicotinamide concentrations was tested (FIG. 1B), it was found that three weeks culture with all concentrations of nicotinamide from 0.5 mM to 5 mM enhanced NK cell proliferation, up to 2.5 times that of the same cells incubated with growth factors ("cytokines") (including IL-2 and IL-15) alone.

Nicotinamide was also able to enhance proliferation of NK cells from a mixed, unselected cord blood mononuclear cell population. Unselected cord blood mononuclear cell cultures were supplemented with 10ng/ml Flt-3, 20ng/ml interleukin-15 (IL-15), 5 ng/ml interleukin-2 (IL-2), with or without 0.5, 1, 2.5 and 5mM nicotinamide. In order to further evaluate the specific effect of nicotinamide on NK cell proliferation *ex-vivo,* analysis of the propagated cells was performed following three weeks in culture, with and without increasing concentrations of nicotinamide. FIGs. 2A and 2B show the clearly dose-dependent nicotinamide-induced enhancement of proliferation in culture of CD56+/CD45+ cells and of CD56+/CD3- NK cells from the mononuclear fraction. FIG. 2C clearly indicates inhibition of CD3+CD56- T cell growth in nicotinamide treated cultures, also in a dose-dependent manner, while CD3+ cells grown in control cultures without nicotinamide predominated over NK cells. Thus, culturing of a mixed cell population of NK and CD3+ (e.g., T and NKT) cells with nicotinamide results in further enrichment of the NK cell compartment, while culture with cytokines only, without nicotinamide, hardly supports NK cell proliferation but enhances the proliferation of CD3+ cells.

Bone marrow and peripheral blood cells were found to contain heterogeneous populations of CD56+ cells containing both CD56+CD3+ NKT and CD56+CD3- NK cells populations. Proliferation of bone-marrow derived NK cells was similarly enhanced by incubation with nicotinamide. Characterization of the inoculated bone marrow purified CD56+ cells shows that about 20-60 % of the cells display the phenotype of NKT cells (CD56+CD3+) and 40-80 % display the phenotype of NK cells (CD56+CD3-) (variable between different BM samples). Therefore, BM derived CD56+ cells comprise a mix population of NK and NKT cells. While proliferation of NK cells in bone marrow derived CD56+ cells incubated with growth factors ("cytokines") (including FLT3, IL-2 and IL-15) alone hardly increased after 14 days in culture, proliferation of NK cells in CD56+ cells incubated with growth factors and 2.5 mM nicotinamide increased 50 % between 14 days (fold increase = 8) and 21 days culture (fold increase = 12)(FIG. 3). Further analysis of CD56+CD3- and CD56+CD3+ subsets of the cultured cells at 21 days revealed overwhelming dominance (greater than 80 %) of CD56+CD3- NK cells over CD56+CD3+ NKT cells in the nicotinamide treated cultures, while CD56+ cells from bone marrow incubated with growth factors ("cytokines") (including FLT3, IL-2 and IL-15) alone were predominantly NKT cells (CD56+CD3+) and not NK (CD56+CD3-)(FIGs. 4A and 4B). Thus, culture of heterogeneous NK + NKT cell populations with nicotinamide results in further propagation and enrichment of the NK cell compartment, with minimal proliferation of NKT cells, while culture with cytokines only without nicotinamide do not support preferential proliferation of NK cells.

The CD56+CD16+ cytotoxic NK cell population has been demonstrated capable of antibody-dependent cell-mediated cytotoxicity (ADCC). Analysis of CD56+CD16+ cells from bone marrow CD56+ cells cultured with growth factors, with and without 2.5 mM nicotinamide after 14 and 21 days in culture revealed significant increase in the fraction of NK cells displaying the CD56+CD16+ phenotype over bone marrow derived CD56+ cells incubated with growth factors ("cytokines") (including FLT3, IL-2 and IL-15) alone (see FIG. 5).

When bone marrow derived CD56+ cells were cultured *ex-vivo* with growth factors ("cytokines") (including FLT3, IL-2 and IL-15) and nicotinamide for 7 days, enhancement of proliferation of the CD56+CD3- NK cell subset (FIG. 6A), and decreased proliferation of CD56+CD3+ NKT cells (FIG. 6B) was evident at ImM, 2.5 mM and 5 mM nicotinamide. Proliferation of CD56+CD16+ NK cells at 7 days was most enhanced with 2.5 mM and 5 mM nicotinamide (FIG. 6C).

### Nicotinamide and the Self Renewal Capacity of Cultured Peripheral Blood NK Cells:

When peripheral blood mononuclear cells are depleted of the CD3+ or CD3+/CD19+ populations, a population enriched in NK cells is obtained, comprising 2-10 % NK cells, with the majority of seeded cells belonging to the myeloid cell lineages. Following 2-3 weeks in culture with nicotinamide, however, more than 90 % of the cells in culture were NK (CD56+CD3-) cells.

Figure 10 illustrates the expansion of peripheral blood NK (T-cell depleted) cells during a three week culture period, in the presence of cytokines (IL-2 or IL-2+IL-15), in scaled-up (10 ml volumes in culture bags) cultures. The expansion was slower in all groups during the first 7 days in culture. After 14 and 21 days, NK expansion was significantly higher in cultures treated with both 2.5 mM and 5 mM nicotinamide relative to control cultures grown without nicotinamide (NAM 0). Intriguingly, NK cells grown with nicotinamide continue to proliferate from day 14 to day 21 while NK cells treated without NAM ceased to proliferate.

When cultures were initiated with a range of seeding densities (2-, 5-, and 10X10⁵ cells seeded), profound enhancement of CD56+CD3- NK cell proliferation was observed at both 2.5 mM and 5 mM after 21 days (Figure 11). FACS analysis of the NK cell cultures at 21 days post seeding shows the clear advantage, for the NK (CD56+/CD3-) population, of culture with nicotinamide at both 2.5 and 5 mM. Note that even though the percentage of NK cells increased all groups, the non-NK cell fraction in cultures treated with nicotinamide is smaller, as compared to the control cultures without nicotinamide (Figure 12).

### Nicotinamide Concentration and Expansion of Cord Blood-Derived. Purified CD56+ NK Cells

When a cord-blood derived NK cell fraction, which had been purified on immunomagnetic beads for the CD56+ phenotype, was cultured with cytokines (including Flt-3, IL-2 and IL-15) alone (cytokines) or in the presence of increasing concentrations (NAM 1 to 7.5 mM) of nicotinamide for up to 3 weeks, it was observed that all concentrations of nicotinamide tested are effective in enhancing NK proliferation in the cord blood derived NK cells (see Figure 17), compared to the cytokine-only controls. Note the dose-dependent increase in the expansion of the cultures exposed to nicotinamide, continuing throughout the entire culture period, compared to controls (cytokines). Thus, nicotinamide's enhancement of NK cell proliferation is effective for short term as well as long term *ex-vivo* NK culture, does not require a feeder layer or feeder cells, is apparent throughout a range of nicotinamide concentrations, and can use either NK-rich, a mixture of NK and non-NK (CD3+, T cells, NKT cells, etc.) cells or highly heterogeneous mononuclear cell populations as a source, from a variety of starting cell populations (e.g., peripheral blood, cord blood), all important for providing large numbers of NK cells for therapeutic use.

### EXAMPLE II- Ex-vivo exposure to nicotinamide enhances NK cell function

NK cells are characterized by response to both inhibitory and activating stimuli, and the production of functional NK cells with effective yet specific cytotoxicity is critical to any considerations of *ex-vivo* NK cell culture. Nicotinamide's effect on NK cell function was assessed by its effect on cell markers, and tested using the chemotactic "Transwell" migration and target cell "killing" assays. In order to detect changes in the prevalence of inhibitory and activating NK cell fractions, purified cord-blood derived CD56+ cells were cultured in wells with growth factors [10ng/ml Flt-3, 20ng/ml interleukin-15 (IL-15), and 5ng/ml interleukin-2 (IL-2)], with or without 1, 2.5 and 5mM nicotinamide. Following 3 weeks culture a significant and dose dependent reduction in the prevalence of the inhibitory CD56+NKG2A cell fraction among NK cells was detected in all nicotinamide concentrations, as compared with cord blood-derived NK cells incubated with growth factors (including FLT3, IL-2 and IL-15) alone (FIG. 7), suggesting enhanced activation of the NK cells with nicotinamide.

Functionally competent NK cells can be characterized by their chemotaxis and cytotoxicity potential. In order to evaluate the effect of nicotinamide on NK cell function, *in-vitro* migration (chemotaxis) and killing (cytotoxicity) assays were performed.

Migration of immunomagnetic purified cord-blood derived CD56+ cells cultured with growth factors and 2.5 or 5 mM nicotinamide, in response to 250 ng/ml SDF stimulus, was greatly enhanced, compared to migration of CD56+ cells cultured with growth factors alone (FIG. 8A). In addition, nicotinamide-cultured CD56+ cells displayed enhanced motility in the absence of SDF stimulus.

The effect of nicotinamide on functionally related cell surface receptors was investigated. When expression of migratory (CXCR4), adhesion (CD49e) and trafficking (CD62L) receptors in the cultured cord-blood derived NK cells was analyzed using specific antibodies and FACS analysis, strongly enhanced expression of CXCR4 and CD62L, and elevated expression of CD49e (FIG. 8B) in cells cultured in the presence of nicotinamide compared to controls (cytokines only) strongly suggests an increased ability of the nicotinamide-treated cells to migrate and home to the bone marrow, lymphoid organs and other organs, and could predict superior in vivo homing and activity of NK cells cultured with nicotinamide.

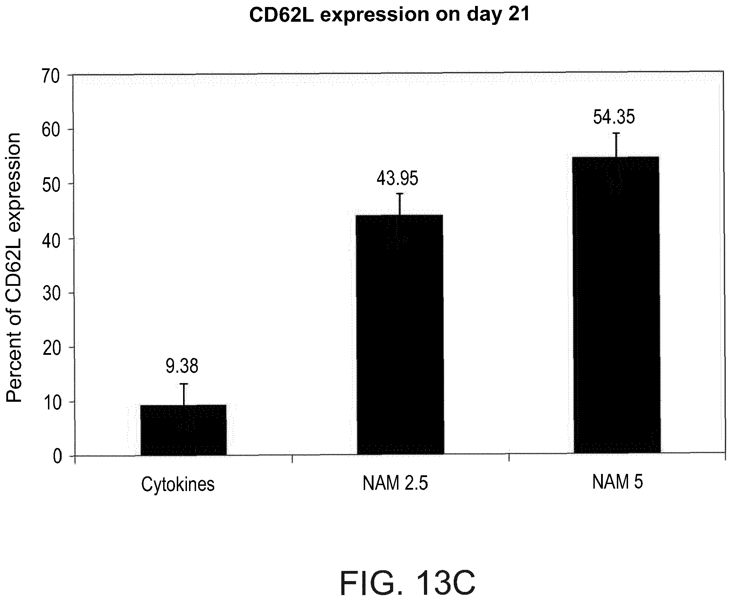

When expression of the trafficking (CD62L) receptor on the surface of T-cell depleted (CD3 or CD3/CD19 depleted) peripheral blood NK cell populations cultured in the presence of nicotinamide was analyzed, elevated expression of this receptor, compared to control cells (cytokines) was observed. At both 2.5 and 5.0 mM nicotinamide, increased percentage of CD62L-positive cells among the NK cells was observed after 7 (Figure 13A), 14 (Figure 13B) and 21 (Figure 13C) days in culture, while the percent of NK cells expressing CD62L in the cytokine-only controls dwindled from 20 % after 7 days to less than 10 % after 21 days in culture.

Figure 20 shows the level of CD62L trafficking receptor expression on peripheral blood-derived, immunobeads-purified CD56+ cells before activation in culture with IL-2, the dramatic decrease in the level of CD62L expression following activation with IL-2 and the remarkable increase in the expression of CD62L following activation in culture with IL-2 and increasing concentrations of nicotinamide (2.5 - 7.5 mM). Cultures were initiated with immunomagnetic beads purified peripheral-blood-derived CD56+ cells and maintained in the presence of cytokines only (including IL-2 and IL-15)(see Figure 20, column "0") or cytokines plus nicotinamide (2.5, 5 and 7.5 mM). Before culture, and after 3 weeks in culture, NK cells were stained with specific CD62L antibodies, and then monitored by FACS. Note the dramatically enhanced expression of CD62L in cells cultured in the presence of nicotinamide compared to controls (cytokines only, "0"). Inset is the FACS data, showing the distribution of CD62L plotted against that of CD56.

Killing potential of NK cells, assayed using CFSE-labeled K562 target cells, was assessed for cord blood-derived CD56+ cells cultured with growth factors with and without nicotinamide. At E:T ratios of 1:5, 1:10 and 1:20 NK cells per target, the killing (cytotoxic) potential of cord blood-derived NK cells cultured with growth factors and nicotinamide was far greater than that of the same number of cells cultured in growth factors only (FIG. 9). Fresh, uncultured cells displayed nearly no killing potential.

Killing potential of peripheral blood NK cells was assayed using CFSE-labeled K562 and BL2 target cells, was assessed for peripheral blood-derived CD56+ cells cultured with growth factors with and without 2.5 mM or 5.0 mM nicotinamide for 3 weeks. Target cell death was monitored by FACS as a percentage of PI-positive CFSE-labeled cells. At E:T ratios from 1:1 to 10:1 NK cells per target, the killing (cytotoxic) potential of the expanded peripheral blood-derived NK cells cultured with growth factors and nicotinamide was far greater than that of the same number of cells cultured in growth factors only (Figure 15A).

Cytotoxic potential of expanded NK cells from peripheral blood was also demonstrated using human primary bi-phenotypic leukemia cells (in which > 90 % of the cells are CD3+ T-cells) as target cells. Figures 15B and 15C shows the results, after 24 hours, using NK cells of 2 separate donors (Donor A and Donor B), indicating increased activation of NK cells killing potential by culturing with nicotinamide. The absence of any killing potential in the cultured NK control cells from Donor A is a further indication of the strong activating effect of nicotinamide on the NK cells.

Cytotoxic potential of NK cells from peripheral blood expanded with nicotinamide towards solid tumor cells, an important therapeutic target, was demonstrated using cells of the colo205 colorectal adenocarcinoma cell line as target cells. Figure 15D shows increased killing potential by NK cells culturing with 5 mM nicotinamide at all of the E:T ratios tested (1:1 to 10:1).

Taken together, these results indicate that exposure of CD56+ cells to nicotinamide not only increases proliferation of NK cells, and specifically proliferation of the CD56+CD62L cell population, but that cells propagated in the presence of nicotinamide have greater motility, directed migration and cytotoxic potential than similar cells cultured with growth factors alone, without nicotinamide. Further, the results shown herein indicate that nicotinamide is effective for enhancing proliferation and functionality of NK cells whether purified or from total mononuclear cell fraction, and from various sources, such as peripheral blood, bone marrow or cord blood.

### Example III- Effect of nicotinamide on proliferation of NK cells cultured with feeder cells

NK cells can also be cultured with feeder cells. The effect of nicotinamide on proliferation and functionality of NK cells was assessed in co-culture with peripheral blood mononuclear cell (PBMC) feeder cells.

In general, culture with feeder cells is carried out as follows: The NK-containing cell populations (e.g., cord blood, peripheral blood, bone marrow) are cultured in the presence of irradiated allogeneic or autologous PBMC feeder cells at a ratio of 10:1 - 100:1 feeder:cultured cells, with a combination of cytokines including FLT3, IL-2 and/or IL-15 and increasing concentrations of nicotinamide (0.5-10mM). The cultures are maintained for 2-5 weeks and the effect of nicotinamide in feeder cell cultures assessed based on distribution of NK and non-NK cells and sub-sets (e.g., CD56+, CD3+, CD56+CD3-, CD56+CD16+) and function of NK cells (e.g., chemotaxis, cytotoxicity). Increased proliferation and functionality of NK cells cultured with nicotinamide in feeder cell cultures indicates further utility of nicotinamide for propagation of NK cells in culture.

In order to assess the effect of nicotinamide on NK cell culture with feeder cells, peripheral blood NK cell fractions were expanded in co-culture with irradiated stroma cells, with and without nicotinamide, and assayed for fold expansion, surface markers CD56, CXCR4 and functional performance.

Peripheral blood-derived mononuclear cell fractions were CD3 depleted and then selected for CD56+ cells by immunobead purification. A portion of the mononuclear cells from the same blood unit was always retained, and irradiated at 3000 rad, to provide irradiated mononuclear cells. The CD56+ NK cells were seeded with irradiated peripheral blood-derived mononuclear cell fractions, at a ratio of irradiated peripheral blood mononuclear cells to CD56 selected cells of 10:1. Cultures were supplemented with IL-2, IL-15 and Flt-3 with or without nicotinamide (2.5 and 5 mM).

After 21 days in culture with the irradiated feeder cells, a clear advantage to the nicotinamide-supplemented cultures was discerned. Fold increase, at day 21, of the NK cells cultured in the presence of both 2.5 mM and 5 mM nicotinamide was at least 4 times that of the control (cytokines + irradiated mononuclear cells), relative to day 0 (see Figure 20). Co-culture of the NK cells with irradiated mononuclear cells, in the presence of nicotinamide, also produced a marked increase in the expression of surface markers CXCR4 (see Figure 21) and CD62L (see Figure 22). Thus, NK cells expanded with nicotinamide, in the presence of irradiated mononuclear cells and cytokines demonstrated enhanced migratory (CXCR4) and trafficking (CD62L) potential.

When the expanded NK cells were assayed for *ex-vivo* killing potential of K562 target cells, increased cytotoxic capability was observed at 5:1, 2.5:1 and 1:1 NK cells per target cell (see Figure 23). Target cell death was monitored by FACS as a percentage of PI-positive CFSE-labeled cells. The increased target cell killing potential, compared to control (cytokines only) strongly suggests enhanced activation of NK cells killing potential by culture with nicotinamide and irradiated mononuclear cells. Thus, the same enhancement of proliferation and functionality were observed when NK cells were cultured in the presence of nicotinamide, with or without additional feeder cells.

Taken together, these results indicate the effects observed in NK cell culture treated with nicotinamide are distinct and greater than the effects of culturing NK cells with feeder cells. Culturing NK cells with nicotinamide, with or without feeder cells, not only resulted in their increased *ex-vivo* proliferation, but also enhanced functional potential (e.g., CD62L and CXCR4 expression) of the expanded NK cells, important for transplantation and clinical efficacy of adoptive immunotherapy treatment with NK cells.

### Example IV- Effect of nicotinamide on proliferation of non-NK cells in NK-cell cultures initiated with CD3- or CD3CD19- depleted MNC

Methods for expansion of NK cells in culture, for use in the clinical setting, must be somewhat specific for the NK cell population, lest contaminating non-NK cells also proliferate due to cell culture conditions. The effect of nicotinamide on non-NK cells was evaluated by monitoring monocyte and granulocyte populations in NK cell cultures exposed to 2.5, 5 and 7.5 mM nicotinamide.

Figures 14A and 14B illustrates the effect of nicotinamide in lowering the percentages of contaminating monocyte (CD14+, Figure 14A) and granulocyte (CD15+, Figure 14B) cells in 14 day NK cell cultures treated with nicotinamide as compared to their percentages in NAM non-treated cultures. Surprisingly, culture of the NK cells with nicotinamide resulted in greater than 50 % suppression of CD14 and CD16 proliferation at 14 days culture.

When the proportion of different lymphocyte subsets, grouped according to cell surface markers (CD56, CD3) present in cultures initiated with CD56+ purified peripheral blood NK cells, and maintained in the absence (cytokines, "NAM 0") or presence of nicotinamide (NAM 2.5, NAM 5.0) was assayed three weeks after initiation, a reduction in fraction of non-NK cells, such as CD3+ T cells and CD3+CD56+ NKT cells, was observed with increasing nicotinamide concentration (see Figure 18 and Figure 18B, inset). NK cells cultured with nicotinamide inhibited CD3+ cells with greater efficiency than NK cells not exposed to nicotinamide. Cultures were maintained with cytokines (including Flt-3, IL-15 and IL-2), with or without increasing concentrations (2.5 to 7.5 mM) of nicotinamide for 3 weeks, reacted with specific antibodies for surface markers, and then analyzed by FACS for the specific phenotypes.

This reduction of contamination from CD14 and CD15 cells, which are often associated with graft versus host disease, NKT and T-cells, can be an important aspect of any clinically useful protocol for expansion of NK cells in culture, as it can reduce the need for extensive T-cell, monocyte and granulocyte depletion pre-implantation, and further decrease graft versus host disease and related complications in the host.

### Example V- Effect of nicotinamide on NK proliferation and function in culture initiated with purified hematopoietic stem/progenitor cells

Nicotinamide enhancement of NK cell proliferation and functionality in hematopoietic-derived cells can be evaluated by further exposure of nicotinamide-treated hematopoietic cell populations to nicotinamide and appropriate growth factors in culture.

Cultures are initiated with purified hematopoietic stem and/or progenitor cells (e.g., CD34+ or CD133+ cells), and supplemented with a combination of cytokines including TPO, FLT3, SCF and/or IL7 and or IL15 for one or two weeks, with or without increasing concentrations of nicotinamide (0.5-10mM). After this period the cultures are supplemented for an additional period (e.g., 2-3 weeks) with a combination of cytokines including FLT3, IL7, IL15, IL21 and/or IL-2, with and without increasing concentrations of nicotinamide (0.5-10mM). In order to evaluate the effect of nicotinamide on proliferation and functionality of the cultured hematopoietic-derived NK cells, distribution of NK and non-NK cells and sub-sets (e.g., CD56+, CD3+, CD56+CD3-, CD56+CD16+) and function of NK cells (e.g., chemotaxis, cytotoxicity) is monitored in the cultured cell populations.

### EXAMPLE VI- Engraftment and therapeutic potential of nicotinamide-cultured NK cells

NK cell expanded in the presence of nicotinamide were tested for localization to target organs and engraftment into the organs *in-vivo* following transplantion of the NK cells into living hosts.

Irradiated (350 Rad) NOD/SCID mice received 15X10⁶ cells from human peripheral blood NK (T-cell depleted) cultures maintained for up to 3 weeks with (NAM 2.5mM and NAM 5mM) or without (NAM 0) nicotinamide. Upon sacrifice of the mice 4 days post infusion, samples of spleen, bone marrow and peripheral blood were evaluated by FACS for the presence of human NK cells (CD56+CD45+) using human specific Abs. Figure 16 shows the increased localization and engraftment into the appropriate target tissues of the NK cells expanded with nicotinamide, expressed as percentage of the total NK cells from the indicated organ, with the higher concentration of nicotinamide demonstrating a stronger effect. Thus, culture of the NK cells with nicotinamide not only increases the numbers of the NK cells, but serves to increase their *in-vivo* functional potential, as demonstrated by localization and engraftment into the target organs (e.g., spleen, bone marrow and peripheral blood).

In further studies, transfusion of cultured, as well as fresh, uncultured human NK or CD56+ cells can be performed, over a range of doses intended to achieve a sub-optimal transplantation, and subsequent non-engraftment in a fraction of mice or their progeny. Human NK cell homing and retention can be evaluated 4 hours and up to 4-weeks post transplantation, for example, according to human CD45 or CD45CD56 antigens. Using the sub-optimal dose, nicotinamide-cultured NK cells can be administered, and the effect on homing and retention in the PB, BM and lymphatic organs of the recipients can recorded, and compared with homing and retention of NK cells cultured without nicotinamide (cytokines only) or fresh NK cells.

The *in-vivo* anti-tumor potential of NK cells cultured with nicotinamide can be assessed using a number of animal tumor models such as myelogenous leukemia (K562) Burkitt's lymphoma (BL-2), human pancreatic cancer (BxPC-3 and Panc-1), human breast cancer and colorectal cancer xenotransplants - growth of xenotransplanted solid tumors is monitored at different time points following infusion of NK cells population of the invention. Metastatic potential of xenotransplanted tumors is also assessed following NK cell infusion. The effect of NK cells on leukemia and myelodysplastic syndrome models can be evaluated directly, using infusion of 1X10³, 1X10⁴, 1X10⁵, 1X10⁶, 1X10⁷, 1X10⁸ or greater cultured NK cells per kg mouse, or evaluated for improving efficacy of post-ablation bone-marrow repopulation when infused along with hematopoietic progenitors, bone marrow, stem cells and the like.

In addition, citation or identification of any reference in this application shall not be construed as an admission that such reference is available as prior art to the present invention. To the extent that section headings are used, they should not be construed as necessarily limiting.

## Claims

1. A method of *ex-vivo* culturing natural killer (NK) cells, the method comprising culturing a population of T-cell depleted cells comprising NK cells with IL-15 and exposing said NK cells to 1 to 10 mM nicotinamide for a duration of exposure of 12-23 hours, wherein culturing said NK cells with IL-15 and said exposing to said nicotinamide results in: (a) elevated expression of CD62L as compared to T-cell depleted NK cells cultured under otherwise identical culturing conditions with less than 0.1 mM of said nicotinamide, and
(b) greater cytotoxicity as compared to T-cell depleted NK cells cultured under otherwise identical culturing conditions with less than 0.1 mM of said nicotinamide.

2. The method of claim 1, wherein said at least one growth factor is IL-15, said culturing with IL-15 is for 14 days from seeding and said concentration of said nicotinamide is 5-10 mM.

3. The method of claim 1 or 2, wherein said exposure to said nicotinamide is from seeding.

4. The method of any one of claims 1-3, wherein said NK cells are exposed to said nicotinamide overnight.

5. The method of any one of claims 1-4, wherein said population of cells comprising said NK cells is obtained from a source selected from the group consisting of cord blood, bone marrow and peripheral blood.

6. The method of any one of claims 1-5, wherein said population of T-cell depleted cells comprising said NK cells is a mononuclear or total nuclear cell population depleted of CD3+ cells and optionally CD3+ and CD19+ cells.

7. The method of any one of claims 1-6, wherein said culturing is without feeder cells.

8. A method of transducing *ex-vivo* cultured NK cells with an exogene, the method comprising:
(a) *ex-vivo* culturing a population of NK cells according to the method of any one of claims 1 to 7; and
(b) transducing said cultured population of NK cells with the exogene.

## Patentansprüche

1. Verfahren zur *Ex-vivo*-Kultivierung natürlicher Killerzellen (NK-Zellen), wobei das Verfahren ein Kultivieren einer Population von an T-Zellen abgereicherten Zellen, die NK-Zellen mit IL-15 umfasst, und ein Einwirkenlassen von 1 bis 10 mM Nicotinamid auf die NK-Zellen für eine Einwirkdauer von 12-23 Stunden umfasst, wobei das Kultivieren der NK-Zellen mit IL-15 und das Einwirkenlassen des Nicotinamids zur Folge hat: (a) erhöhte Expression von CD62L, verglichen mit an T-Zellen abgereicherten NK-Zellen, die unter ansonsten identischen Kulturbedingungen mit weniger als 0,1 mM des Nicotinamids kultiviert werden, und
(b) höhere Zytotoxizität, verglichen mit an T-Zellen abgereicherten NK-Zellen, die unter ansonsten identischen Kulturbedingungen mit weniger als 0,1 mM des Nicotinamids kultiviert werden.

2. Verfahren nach Anspruch 1, wobei der wenigstens eine Wachstumsfaktor IL-15 ist und das Kultivieren mit IL-15 für 14 Tage ab dem Ansetzen erfolgt und die Konzentration des Nicotinamids 5-10 mM beträgt.

3. Verfahren nach Anspruch 1 oder 2, wobei das Einwirkenlassen des Nicotinamids ab dem Ansetzen erfolgt.

4. Verfahren nach einem der Ansprüche 1-3, wobei das Einwirken des Nicotinamids auf die NK-Zellen über Nacht erfolgt.

5. Verfahren nach einem der Ansprüche 1-4, wobei die Zellpopulation, welche die NK-Zellen umfasst, aus einer Quelle erhalten wird, die aus der Gruppe bestehend aus Nabelschnurblut, Knochenmark und peripherem Blut ausgewählt ist.

6. Verfahren nach einem der Ansprüche 1-5, wobei die Population von an T-Zellen abgereicherten Zellen, welche die NK-Zellen umfasst, eine mononukleäre oder gesamte nukleäre Zellpopulation ist, die an CD3+-Zellen und optional CD3+- und CD19+-Zellen abgereichert ist.

7. Verfahren nach einem der Ansprüche 1-6, wobei das Kultivieren ohne Fütterzellen erfolgt.

8. Verfahren zur Transduktion von *ex vivo* kultivierten NK-Zellen mit einem exogenen Gen, wobei das Verfahren umfasst:
(a) *Ex-vivo*-Kultivieren einer Population von NK-Zellen gemäß dem Verfahren nach einem der Ansprüche 1 bis 7; und
(b) Transduzieren der kultivierten Population von NK-Zellen mit dem exogenen Gen.

## Revendications

1. Procédé de culture *ex vivo* de cellules tueuses naturelles (NK), le procédé comprenant cultiver une population de cellules appauvries en cellules T comprenant des cellules NK avec IL-15 et exposant lesdites cellules NK à de 1 à 10 mM de nicotinamide pendant une durée d'exposition de 12-23 heures, cultiver desdites cellules NK avec IL-15 et ladite exposition au susdit nicotinamide entraînant : (a) une expression accrue de CD62L comparativement aux cellules NK appauvries en cellules T cultivées dans des conditions de culture par ailleurs identiques avec moins de 0,1 mM du susdit nicotinamide, et
(a) une cytotoxicité accrue comparativement aux cellules NK appauvries en cellules T cultivées dans des conditions de culture par ailleurs identiques avec moins de 0,1 mM du susdit nicotinamide.

2. Procédé selon la revendication 1, l'au moins un facteur de croissance étant IL-15, ladite culture avec IL-15 étant pendant 14 jours à partir de l'ensemencement et ladite concentration en susdit nicotinamide étant de 5-10 mM.

3. Procédé selon la revendication 1 ou 2, ladite exposition au susdit nicotinamide étant à partir de l'ensemencement.

4. Procédé selon l'une quelconque des revendications 1-3, lesdites cellules NK étant exposées au susdit nicotinamide pendant la nuit.

5. Procédé selon l'une quelconque des revendications 1-4, ladite population de cellules comprenant lesdites cellules NK étant obtenue à partir d'une source sélectionnée parmi le groupe se composant de : sang de cordon ombilical, moelle osseuse et sang périphérique.

6. Procédé selon l'une quelconque des revendications 1-5, ladite population de cellules appauvries en cellules T comprenant lesdites cellules NK étant une population de cellules mononucléaires ou nucléaires totales appauvries en cellules CD3+ et alternativement en cellules CD3+ et CD19+.

7. Procédé selon l'une quelconque des revendications 1-6, ladite culture étant sans cellules nourricières.

8. Procédé de transduction de cellules NK cultivées *ex vivo* avec un gène exogène, le procédé comprenant :
(a) culture *ex vivo* d'une population de cellules NK conformément au procédé selon l'une quelconque des revendications 1 à 7 ; et
(b) transduction de ladite population cultivée de cellules cultivée avec le gène exogène.
